# EUROPEAN PATENT SPECIFICATION

(11) **EP 3 218 059 B1**
(45) Date of publication and mention of the grant of the patent: **06.01.2021**
(21) Application number: 15858728.7
(22) Date of filing: 13.11.2015
(51) Int. Cl.: A61K 31/16, A61K 31/165, A61P 17/02, A61P 29/00

(54) **CAPSAICINOIDS FOR USE IN TREATING ACRAL LICK GRANULOMA**
CAPSAICINOIDE ZUR VERWENDUNG BEI DER BEHANDLUNG VON AKRALEM LECKGRANULOM
CAPSAÏCINOÏDES UTILISÉS DANS LE TRAITEMENT DU GRANULOME PAR LÉCHAGE DES EXTRÉMITÉS

(30) Priority: 13.11.2014 US 201462079121 P
(43) Date of publication of application: 20.09.2017
(73) Proprietor: Centrexion Therapeutics Corporation, Baltimore, MD 21202 (US)
(72) Inventor: CAMPBELL, James N., Baltimore, MD 21230 (US); HANSON, Peter D., Prides Crossing, MA 01965 (US)
(74) Representative: Graham Watt & Co LLP
(86) International application number: PCT/US2015/060670
(87) International publication number: WO 2016/077749

(56) References cited:
- WO-A1-2004/058286
- Anonymous: "Acral lick dermatitis", , 19 December 2008 (2008-12-19), pages 1-2, XP055460033, Retrieved from the Internet: URL:http://robinsonvet.com/documents/Acral LickDermatitis.pdf [retrieved on 2018-03-16]
- DRAY, A ET AL.: 'Mechanism of action of capsaicin-like molecules on sensory neurons.' LIFE SCIENCE vol. 51, no. 23, 01 January 1992, pages 1759 - 1765, XP000576700 DOI: 10.1016/0024-3205(92)90045-Q
- WINTER, J ET AL.: 'Capsaicin and pain mechanisms.' BRITISH J OF ANAESTHESIA vol. 75, 01 January 1995, pages 157 - 168, XP003034340
- AASVANG, E K ET AL.: 'Late sensory function after intraoperative capsaicin wound instillation.' ACTA ANAESTHESIOLOGICA SCANDINAVICA vol. 54, 01 January 2010, pages 224 - 231, XP055440788 DOI: 10.1111/J.1399-6576.2009.02068.X
- "Canine and Feline Dermatology Drug Handbook", 1 January 2012 (2012-01-01), Wiley Blackwell pages 364-364,

## Description

### FIELD OF THE INVENTION

The invention provides formulations for use in treating acral lick granuloma in a veterinary animal, such as a canine, using a capsaicinoid such as capsaicin.

### BACKGROUND

Acral lick granuloma is a form of self-trauma and skin disorder in which animals, particularly dogs, continuously lick a small area of their body until it becomes raw and inflamed. Certain reports have described acral lick granuloma as a psychogenic disorder with corollaries to obsessive compulsive disorder. Once a lesion is present, pain and/or itch associated with the irritation may perpetuate the licking behavior. Also, the lesion may develop secondary problems, such as a bacterial infection, ruptured apocrine glands, and ruptured hair follicles.

Various reports indicate that dogs of all breeds and both sexes are susceptible to this disease. Some evidence suggests that male dogs or dogs five years of age or older are disproportionately affected. Furthermore, certain dog breeds may be predisposed to acral lick granuloma, including Golden Retrievers, Labrador Retrievers, Great Danes, Doberman Pinschers, Irish Setters, and German Shepherds.

Currently available treatments for acral lick granuloma can involve preventing licking through the use of Elizabethan collars, muzzles, bandages, or noxious ointments, antibiotic therapy if a bacterial infection is present, and decreasing inflammation through the local use of corticosteroids. Other treatment approaches that are currently available include radiation therapy, laser surgery, cryotherapy, acupuncture, and administration of antihistamines. However, acral lick granuloma remains difficult to treat using these prior approaches.
Anonymous, "Acral lick dermatitis", (20081219), pages 1 - 2, URL: http://robinsonvet.com/documents/AcralLickDermatitis.pdf, relates to the treatment of acral lick dermatitis.

Accordingly, a need exists for improved treatments for acral lick granuloma. The present invention addresses this need and provides other related advantages.

### SUMMARY

The invention provides liquid capsaicin injectable formulations for use in treating acral lick granuloma in a canine, according to the claims. Methods are described for deactivating a nerve fiber selected from the group consisting of a C-fiber and an A-delta fiber, each being located in or proximal to an acral lick granuloma in a veterinary animal, such as a canine, using a capsaicinoid such as capsaicin. Various aspects and embodiments of the invention are described in further detail below.

Accordingly, a method is described of treating acral lick granuloma in a canine. The method comprises administering to a canine in need thereof a therapeutically effective amount of a capsaicinoid, such as capsaicin, to the granuloma or tissue proximal thereto, to thereby treat the acral lick granuloma. The capsaicinoid is preferably administered by subcutaneous injection. The method preferably provides relief from itch and/or pain due to the acral lick granuloma for a duration of at least 2 weeks, or at least 4 weeks.

A method is described of treating acral lick granuloma in a canine, where the method comprises administering to a canine in need thereof a therapeutically effective amount of a liquid capsaicin injectable formulation by performing multiple subcutaneous injections of the liquid capsaicin injectable formulation to tissue proximal to the lower boundary of the granuloma, wherein, after the first subcutaneous injection of the formulation to tissue proximal to the lower boundary of the granuloma, any subsequent subcutaneous injection of the formulation into tissue proximal to the lower boundary of the granuloma is performed at a location laterally distal to any prior injection of the formulation to tissue proximal to the lower boundary of the granuloma, to thereby disperse capsaicin throughout the area under the acral lick granuloma in proximity to the boundary between healthy tissue and the granuloma; wherein the administering delivers capsaicin in an amount ranging from about 1.2 µg to about 1.8 µg of capsaicin per square millimeter of topical surface area of the acral lick granuloma; the liquid capsaicin injectable formulation contains capsaicin in an amount ranging from about 0.3 mg/mL to about 0.5 mg/mL; and the capsaicin comprises greater than about 95% trans-capsaicin, to thereby treat the acral lick granuloma. Preferably, the administering delivers capsaicin in an amount of about 1.5 µg of capsaicin per square millimeter of topical surface area of the acral lick granuloma. Also preferably, the multiple subcutaneous injections of a liquid capsaicin injectable formulation to tissue proximal to the lower boundary of the granuloma are injections of the liquid capsaicin injectable formulation into healthy tissue proximal to the lower boundary of the granuloma. The method may be further characterized according the volume of formulation administered, such as where the administering delivers from about 0.3 mL to about 5 mL of the formulation. In certain embodiments, the method comprises injecting a liquid capsaicin injectable formulation into acral lick granuloma tissue.

A method of deactivating a nerve fiber selected from the group consisting of a C-fiber and an A-delta fiber, each being located in or proximal to an acral lick granuloma in a canine is described. The method comprises administering to a canine in need thereof an effective amount of a capsaicinoid to the granuloma or tissue proximal thereto, to thereby deactivate said nerve fiber. The capsaicinoid is preferably administered by subcutaneous injection. The method preferably provides relief from itch and/or pain due to the acral lick granuloma for a duration of at least 2 weeks, at least 4 weeks, at least 12 weeks, or at least 16 weeks.

### BRIEF DESCRIPTION OF THE DRAWINGS

**Figure** 1 provides illustrations of the lesion(s) from each dog prior to injection with Study Drug, along with illustrations of the lesions from each dog at Day 14 and separately at Day 28 of the study, as further described in Example 1 herein.
**Figure** 2 provides illustrations of the lesion(s) from each dog prior to injection of Study Drug, at Day 14, and at Day 28 of the study, where the lesion is traced for Adobe Acrobat IX determination of lesion area, as further described in Example 1 herein.

### DETAILED DESCRIPTION

Methods are described for treating acral lick granuloma in a veterinary animal, such as a canine, using a capsaicinoid such as capsaicin. Methods are also described for deactivating a nerve fiber selected from the group consisting of a C-fiber and an A-delta fiber, each being located in or proximal to an acral lick granuloma in a veterinary animal, such as a canine, using a capsaicinoid such as capsaicin. Various aspects of the invention are set forth below in sections; however, aspects of the invention described in one particular section are not to be limited to any particular section.

### I. THERAPEUTIC APPLICATIONS

Methods for treating acral lick granuloma in a veterinary animal, such as a canine, using a capsaicinoid such as capsaicin are described. The method generally comprises administering to a veterinary animal in need thereof a therapeutically effective amount of a capsaicinoid to the granuloma or tissue proximal thereto, to thereby treat the acral lick granuloma. Methods for deactivating a nerve fiber selected from the group consisting of a C-fiber and an A-delta fiber, each being located in or proximal to an acral lick granuloma in a veterinary animal, such as a canine, using a capsaicinoid such as capsaicin, are also described. The method generally comprises administering to a veterinary animal in need thereof an effective amount of a capsaicinoid to the granuloma or tissue proximal thereto, to thereby deactivate said nerve fiber. Various aspects and embodiments of the methods are described below.

### Exemplary Method for Treating Acral Lick Granuloma

A method of treating acral lick granuloma in a canine is described. The method comprises administering to a canine in need thereof a therapeutically effective amount of a capsaicinoid to the granuloma or tissue proximal thereto, to thereby treat the acral lick granuloma.

In certain embodiments, the capsaicinoid is administered to the granuloma. In certain embodiments, the capsaicinoid is administered to the granuloma and tissue proximal to the granuloma. In certain embodiments, the capsaicinoid is administered to tissue proximal to the granuloma. In certain embodiments, the tissue proximal thereto contains a nerve fiber extending into the granuloma.

### Exemplary More Specific Method for Treating Acral Lick Granuloma

A method of treating acral lick granuloma in a canine is described. The method comprises administering to a canine in need thereof a therapeutically effective amount of a liquid capsaicin injectable formulation by performing multiple subcutaneous injections of the liquid capsaicin injectable formulation to tissue proximal to the lower boundary of the granuloma, wherein, after the first subcutaneous injection of the formulation to tissue proximal to the lower boundary of the granuloma, any subsequent subcutaneous injection of the formulation into tissue proximal to the lower boundary of the granuloma is performed at a location laterally distal to any prior injection of the formulation to tissue proximal to the lower boundary of the granuloma, to thereby disperse capsaicin throughout the area under the acral lick granuloma in proximity to the boundary between healthy tissue and the granuloma; wherein the administering delivers capsaicin in an amount ranging from about 1.2 µg to about 1.8 µg of capsaicin per square millimeter of topical surface area of the acral lick granuloma; the liquid capsaicin injectable formulation contains capsaicin in an amount ranging from about 0.3 mg/mL to about 0.5 mg/mL; and the capsaicin comprises greater than about 95% *trans*-capsaicin, to thereby treat the acral lick granuloma.

Preferably, the administering delivers capsaicin in an amount of about 1.5 µg of capsaicin per square millimeter of topical surface area of the acral lick granuloma. Also preferably, the multiple subcutaneous injections of a liquid capsaicin injectable formulation to tissue proximal to the lower boundary of the granuloma are injections of the liquid capsaicin injectable formulation into healthy tissue proximal to the lower boundary of the granuloma. In certain embodiments, the method further comprises injecting a liquid capsaicin injectable formulation into acral lick granuloma tissue.

The method may be further characterized according to the volume of formulation administered, such as where the administering delivers from about 0.3 mL to about 5 mL of the formulation.

The method may be further characterized according to reducing certain canine behaviors indicative of discomfort, such as reducing the amount of licking the acral lick granuloma, biting at the acral lick granuloma, and/or scratching the acral lick granuloma. The method may also be characterized according to healing parameters of the acral lick granuloma, such as the regrowth of hair over tissue previously affected by the acral lick granuloma. Accordingly, in certain embodiments, the administering provides one or more of the following:
- at least a 30% reduction in the number of times the canine engaged in licking the acral lick granuloma on the day that is 28 days after receiving the capsaicin compared to the average number of times the canine engaged in licking the acral lick granuloma per day for a period (e.g., the 7 day period) prior to receiving the capsaicin;
- at least a 30% reduction in the number of times the canine engaged in biting at the acral lick granuloma on the day that is 28 days after receiving the capsaicin compared to the average number of times the canine engaged in biting at the acral lick granuloma per day for a period (e.g., the 7 day period) prior to receiving the capsaicin;
- at least a 30% reduction in the number of times the canine engaged in scratching the acral lick granuloma on the day that is 28 days after receiving the capsaicin compared to the average number of times the canine engaged in scratching the acral lick granuloma per day for a period (e.g., the 7 day period) prior to receiving the capsaicin; or
- hair regrows over at least about 30% of the topical area of the original lesion due to the acral lick granuloma by day twenty-eight after receiving the capsaicin.

The method may be further characterized according to changes in Client-Specific Outcome Measure (CSOM) score, which can reflect relief from canine discomfort due to the acral lick granuloma. Accordingly, in certain embodiments, the administering provides one or more of the following (a) at least a 40% reduction in CSOM score at day 14 after receiving capsaicin compared to the CSOM score observed on a day prior to receiving capsaicin (e.g., the day that was 7 days prior to receiving capsaicin); or (b) at least a 20% reduction in CSOM score at day 28 after receiving capsaicin compared to the CSOM score observed on a day prior to receiving capsaicin (e.g., on the day that was 7 days prior to receiving capsaicin).

The method may be further characterized according to the duration of relief from itch, relief from pain, and/or deactivation of a nerve fiber selected from the group consisting of a C-fiber and an A-delta fiber. Accordingly, in certain embodiments, the administering provides relief from itch due to the acral lick granuloma for a duration of at least 6 weeks. In certain other embodiments, the administering provides relief from pain due to the acral lick granuloma for a duration of at least 6 weeks. In yet other embodiments, the administering provides deactivation of a nerve fiber selected from the group consisting of a C-fiber and an A-delta fiber, each being located in or proximal to an acral lick granuloma in the canine, for a duration of at least 4 weeks.

### Additional Exemplary More Specific Method for Treating Acral Lick Granuloma

Another more specific method is described of treating acral lick granuloma in a canine, where the method comprises administering to a canine in need thereof a therapeutically effective amount of a liquid capsaicin injectable formulation by performing multiple subcutaneous injections of the liquid capsaicin injectable formulation to tissue proximal to the lower boundary of the granuloma, wherein, after the first subcutaneous injection of the formulation to tissue proximal to the lower boundary of the granuloma, any subsequent subcutaneous injection of the formulation into tissue proximal to the lower boundary of the granuloma is performed at a location laterally distal to any prior injection of the formulation to tissue proximal to the lower boundary of the granuloma, to thereby disperse capsaicin throughout the area under the acral lick granuloma in proximity to the boundary between healthy tissue and the granuloma, to thereby treat the acral lick granuloma. In certain embodiments, the administering delivers capsaicin in an amount ranging from about 1.2 µg to about 1.8 µg of capsaicin per square millimeter of topical surface area of the acral lick granuloma. In certain embodiments, the liquid capsaicin injectable formulation contains capsaicin in an amount ranging from about 0.3 mg/mL to about 0.5 mg/mL. In certain embodiments, the capsaicin comprises greater than about 95% *trans*-capsaicin.

Preferably, the administering delivers capsaicin in an amount of about 1.5 µg of capsaicin per square millimeter of topical surface area of the acral lick granuloma. Also preferably, the multiple subcutaneous injections of a liquid capsaicin injectable formulation to tissue proximal to the lower boundary of the granuloma are injections of the liquid capsaicin injectable formulation into healthy tissue proximal to the lower boundary of the granuloma. In certain embodiments, the method further comprises injecting a liquid capsaicin injectable formulation into acral lick granuloma tissue.

The method may be further characterized according to the volume of formulation administered, such as where the administering delivers from about 0.3 mL to about 5 mL of the formulation.

The method may be further characterized according to reducing certain canine behaviors indicative of discomfort, such as reducing the amount of licking the acral lick granuloma, biting at the acral lick granuloma, and/or scratching the acral lick granuloma. The method may also be characterized according to healing parameters of the acral lick granuloma, such as the regrowth of hair over tissue previously affected by the acral lick granuloma.

### Exemplary Method for Deactivating Nerve Fibers

A method of deactivating a nerve fiber selected from the group consisting of a C-fiber and an A-delta fiber, each being located in or proximal to an acral lick granuloma in a canine, is also described. The method comprises administering to a canine in need thereof an effective amount of a capsaicinoid to the granuloma or tissue proximal thereto, to thereby deactivate said nerve fiber.

In certain embodiments, the administering deactivates a C-fiber. In certain embodiments, the administering deactivates an A-delta fiber. In certain embodiments, the administering deactivates C-fibers and A-delta fibers. In certain embodiments, the administering deactivates C-fibers and A-delta nociceptive afferents.

### Exemplary Method for Treating Additional Skin Disorders

A method of treating a lesion of the skin that produces itch in a veterinary animal, such as a canine, is also described. The method comprises administering to a veterinary animal in need thereof a therapeutically effective amount of a capsaicinoid to the lesion or tissue proximal thereto, to thereby treat the lesion of the skin.

### Exemplary Features of the Methods

As noted above, the methods of treating acral lick granuloma and for deactivating nerve fibers can be characterized in various ways. Some of these features are recited above. A more thorough description of such features are provided below.

### Route of Administration

In certain embodiments, the capsaicinoid is administered by injection. In certain embodiments, the capsaicinoid is administered by subcutaneous injection.

The administration technique may be further characterized according to the specific location into which capsaicinoid is administered via subcutaneous injection. For example, in certain embodiments, the method comprises administering a capsaicinoid to tissue proximal to the lower boundary of the granuloma. In certain other embodiments, the administering comprises performing multiple subcutaneous injections of a capsaicinoid to tissue proximal to the lower boundary of the granuloma, wherein, after the first subcutaneous injection of capsaicinoid to tissue proximal to the lower boundary of the granuloma, any subsequent subcutaneous injection of capsaicinoid into tissue proximal to the lower boundary of the granuloma is performed at a location laterally distal to any prior injection of capsaicinoid to tissue proximal to the lower boundary of the granuloma. In certain embodiments, the multiple subcutaneous injections of capsaicinoid to tissue proximal to the lower boundary of the granuloma are injections of capsaicinoid into healthy tissue proximal to the lower boundary of the granuloma.

The administration technique may be further characterized according to the distribution of capsaicinoid throughout tissue in proximity to the acral lick granuloma. For example, in certain embodiments, the administering results in capsaicinoid dispersed throughout the area under the acral lick granuloma in proximity to the boundary between healthy tissue and the granuloma. In certain other embodiments, the administering comprises performing multiple subcutaneous injections of a capsaicinoid to disperse capsaicinoid throughout the area under the acral lick granuloma in proximity to the boundary between healthy tissue and the granuloma.

The administration technique may be further characterized according to the number of subcutaneous injections. For example, in certain embodiments, the administering comprises at least three subcutaneous injections, at least four subcutaneous injections, or at least five subcutaneous injections. In certain embodiments, the administering comprises three, four, or five subcutaneous injections.

The administration technique may be further characterized according injecting capsaicinoid directly into acral lick granuloma tissue. For example, in certain embodiments, the administering comprises injecting capsaicinoid into the acral lick granuloma. This may be characterized as injecting capsaicinoid into the granulomous tissue.

### Capsaicinoids

Exemplary capsaicinoids include, for example, capsaicin, trans-capsaicin, resiniferatoxin, *N*-vanillylnonanamides, *N*-vanillylsulfonamides, *N*-vanillylureas, *N-*vanillylcarbamates, *N*-[(substituted phenyl)methyl]alkylamides, methylene substituted *N-*[(substituted phenyl)methyl]alkanamides, *N*-[(substituted phenyl)methyl]-cis-monosaturated alkenamides, *N*-[(substituted phenyl)methyl]diunsaturated amides, 3-hydroxyacetanilide, hydroxyphenylacetamides, pseudocapsaicin, dihydrocapsaicin, nordihydrocapsaicin anandamide, piperine, zingerone, warburganal, polygodial, aframodial, cinnamodial, cinnamosmolide, cinnamolide, isovelleral, scalaradial, ancistrodial, beta-acaridial, merulidial, and scutigeral.

In certain embodiments, the capsaicinoid is capsaicin. Capsaicin contains a carbon-carbon double bond and can existing as *cis* and *trans*-isomers. In certain embodiments, the capsaicin consists essentially of *trans*-capsaicin. In certain embodiments, the capsaicin comprises greater than about 90% wt/wt *trans*-capsaicin. In certain embodiments, the capsaicin comprises greater than about 95% wt/wt trans-capsaicin. In certain embodiments, the capsaicin comprises greater than about 98% wt/wt *trans*-capsaicin.

In certain embodiments, the capsaicinoid comprises trans-capsaicin. In certain embodiments, the capsaicinoid consists essentially of trans-capsaicin. In certain embodiments, the capsaicinoid is capsaicin comprising greater than about 90% wt/wt *trans*-capsaicin. In certain embodiments, the capsaicinoid is capsaicin comprising greater than about 95% wt/wt trans-capsaicin. In certain embodiments, the capsaicinoid is capsaicin comprising greater than about 97% wt/wt trans-capsaicin. In certain embodiments, the capsaicinoid is capsaicin comprising greater than about 98% wt/wt *trans*-capsaicin. In certain embodiments, the capsaicinoid is capsaicin comprising greater than about 99% wt/wt trans-capsaicin.

### Dosage of Capsaicinoid

In certain embodiments, the capsaicinoid is administered in an amount in the range of about 0.1 mg to about 10 mg. In certain embodiments, the capsaicinoid is administered in an amount in the range of about 0.2 mg to about 10 mg. In certain embodiments, the capsaicinoid is administered in an amount in the range of about 1 mg to about 4 mg. In certain embodiments, the capsaicinoid is administered in an amount of about 2 mg.

In certain embodiments, the aforementioned dosages are for capsaicin. In certain embodiments, trans-capsaicin is administered in an amount in the range of about 0.1 mg to about 10 mg. In certain embodiments, trans-capsaicin is administered in an amount in the range of about 0.2 mg to about 10 mg. In certain embodiments, trans-capsaicin is administered at an amount in the range of about 1 mg to about 4 mg. In certain embodiments, *trans-*capsaicin is administered at an amount of about 2 mg.

The dose of capsaicinoid may also be characterized based on the amount of capsaicinoid relative to the area of the topical surface of the acral lick granuloma. This may be expressed as administration of a certain number of micrograms of capsaicinoid per square millimeter of topical surface area of the acral lick granuloma, such as about 1.5 micrograms of capsaicinoid per square millimeter of topical surface area of the acral lick granuloma, which may be abbreviated as 1.5 µg/mm². Accordingly, in certain embodiments, the capsaicinoid is administered in an amount ranging from about 0.05 µg to about 5 µg, about 0.1 µg to about 4 µg, about 0.5 µg to about 3 µg, about 1.0 µg to about 2.5 µg, about 1 µg to about 2 µg, about 1.25 µg to about 1.75 µg, about 1.3 µg to about 1.7 µg, or about 1.4 µg to about 1.6 µg of capsaicinoid per square millimeter of topical surface area of the acral lick granuloma. In certain embodiments, the capsaicinoid is administered in an amount ranging from about 0.5 µg to about 1 µg, about 0.75 µg to about 1.25 µg, about 1 µg to about 1.5 µg, about 1.25 µg to about 1.75 µg, about 1.5 µg to about 2 µg, about 2 µg to about 2.5 µg, about 2.5 µg to about 3 µg, about 3 jug to about 3.5 µg, about 3.5 µg to about 4 µg, about 4 µg to about 4.5 µg, or about 4.5 µg to about 5 µg of capsaicinoid per square millimeter of topical surface area of the acral lick granuloma. In certain embodiments, capsaicinoid is administered in an amount ranging from about 1.2 µg to about 1.8 µg of capsaicinoid per square millimeter of topical surface area of the acral lick granuloma. In certain preferred embodiments, the capsaicinoid is administered in an amount ranging from about 1.4 µg to about 1.6 µg of capsaicinoid per square millimeter of topical surface area of the acral lick granuloma. In a preferred embodiment, the dosages recited above are for administration by subcutaneous injection of the capsaicinoid.

In certain embodiments, the aforementioned dosages are for capsaicin. In certain embodiments, capsaicin is administered in an amount ranging from about 0.05 µg to about 5 µg, about 0.1 µg to about 4 µg, about 0.5 µg to about 3 µg, about 1.0 µg to about 2.5 µg, about 1 µg to about 2 µg, about 1.25 µg to about 1.75 µg, about 1.3 µg to about 1.7 µg, or about 1.4 µg to about 1.6 µg of capsaicin per square millimeter of topical surface area of the acral lick granuloma. In certain embodiments, capsaicin is administered in an amount ranging from about 1 µg to about 2 µg of capsaicin per square millimeter of topical surface area of the acral lick granuloma. In certain embodiments, capsaicin is administered in an amount ranging from about 1.2 µg to about 1.8 µg of capsaicin per square millimeter of topical surface area of the acral lick granuloma. In certain embodiments, capsaicin is administered in an amount ranging from about 1.4 µg to about 1.6 µg of capsaicin per square millimeter of topical surface area of the acral lick granuloma. In certain embodiments, *trans*-capsaicin is administered in an amount ranging from about 0.05 µg to about 5 µg, about 0.1 µg to about 4 µg, about 0.5 µg to about 3 µg, about 1.0 µg to about 2.5 µg, about 1 µg to about 2 µg, about 1.25 µg to about 1.75 µg, about 1.3 µg to about 1.7 µg, or about 1.4 µg to about 1.6 µg of trans-capsaicin per square millimeter of topical surface area of the acral lick granuloma. In certain embodiments, *trans*-capsaicin is administered in an amount ranging from about 0.5 µg to about 1 µg, about 0.75 µg to about 1.25 µg, about 1 µg to about 1.5 µg, about 1.25 µg to about 1.75 µg, about 1.5 µg to about 2 µg, about 2 µg to about 2.5 µg, about 2.5 µg to about 3 µg, about 3 µg to about 3.5 µg, about 3.5 µg to about 4 µg, about 4 µg to about 4.5 µg, or about 4.5 µg to about 5 µg of trans-capsaicin per square millimeter of topical surface area of the acral lick granuloma. In certain embodiments, trans-capsaicin is administered in an amount ranging from about 1.2 µg to about 1.8 µg of *trans*-capsaicin per square millimeter of topical surface area of the acral lick granuloma. In certain preferred embodiments, *trans*-capsaicin is administered in an amount ranging from about 1.4 µg to about 1.6 µg of trans-capsaicin per square millimeter of topical surface area of the acral lick granuloma. In a preferred embodiment, the dosages recited above are for administration by subcutaneous injection of the capsaicin, such as *trans*-capsaicin.

In certain other embodiments, the capsaicinoid is administered in an amount of about 0.5 µg, 0.75 µg, 1.0 µg, 1.25 µg, 1.3 µg, 1.4 µg, 1.5 µg, 1.6 µg, 1.7 µg, 1.75 µg, 2 µg, 2.25 µg, 2.5 µg, 2.75 µg, 3 µg, 3.25 µg, 3.5 µg, 3.75 µg, 4 µg, 4.25 µg, 4.5 µg, 4.75 µg, or 5 µg/mm² of capsaicinoid per square millimeter of topical surface area of the acral lick granuloma. In certain preferred embodiments, the capsaicinoid is administered in an amount of about 1.5 µg of capsaicinoid per square millimeter of topical surface area of the acral lick granuloma. In preferred embodiments, the dosages recited above are for administration by subcutaneous injection of the capsaicinoid.

In certain embodiments, the aforementioned dosages are for capsaicin. For example, in certain embodiments, capsaicin is administered in an amount of about 0.5 µg, 0.75 µg, 1.0 µg, 1.25 µg, 1.3 µg, 1.4 µg, 1.5 µg, 1.6 µg, 1.7 µg, 1.75 µg, 2 µg, 2.25 µg, 2.5 µg, 2.75 µg, 3 µg, 3.25 µg, 3.5 µg, 3.75 µg, 4 µg, 4.25 µg, 4.5 µg, 4.75 µg, or 5 ng/mm² of capsaicin per square millimeter of topical surface area of the acral lick granuloma. In certain preferred embodiments, capsaicin is administered in an amount of about 1.5 µg of capsaicin per square millimeter of topical surface area of the acral lick granuloma.

In certain embodiments, trans-capsaicin is administered in an amount of about 0.5 µg, 0.75 µg, 1.0 µg, 1.25 µg, 1.3 µg, 1.4 µg, 1.5 µg, 1.6 µg, 1.7 µg, 1.75 µg, 2 µg, 2.25 µg, 2.5 µg, 2.75 µg, 3 µg, 3.25 µg, 3.5 µg, 3.75 µg, 4 µg, 4.25 µg, 4.5 µg, 4.75 µg, or 5 µg/mm² of *trans*-capsaicin per square millimeter of topical surface area of the acral lick granuloma. In certain preferred embodiments, *trans*-capsaicin is administered in an amount of about 1.5 µg of trans-capsaicin per square millimeter of topical surface area of the acral lick granuloma. In a preferred embodiment, the dosages recited above are for administration by subcutaneous injection of trans-capsaicin.

### Injectable Formulations

Various injectable formulations are described in the literature and known to those of skill in the art. The injectable formulation may typically contain water and one or more additional components to render the formulation optimally suited for injection into a subject.

When administering capsaicinoid according to methods described herein, the capsaicinoid is desirably administered in the form of a pharmaceutical composition formulated for injection. In certain embodiments, the pharmaceutical composition formulated for injection is an aqueous pharmaceutical composition.

The capsaicinoid may be dissolved in oils, polyethylene glycol (PEG), propylene glycol (PG), and/or other solvents commonly used to prepare injectable or implantable solutions. Suitable pharmaceutically acceptable vehicles include aqueous vehicles, nonaqueous vehicles, antimicrobial agents, isotonic agents, buffers, antioxidants, suspending and dispersing agents, emulsifying agents, sequestering or chelating agents, and combinations or mixtures thereof. It is appreciated that when one or more solvents are used in the formulations of the invention, they may be combined, e.g., with a pharmaceutically acceptable buffer and may be present in the final formulation, e.g., in an amount ranging from about 10% to about 100%, more preferably from about 20% to about 100%.

Exemplary aqueous vehicles include Sodium Chloride Injection, Bacteriostatic Sodium Chloride Injection, Ringers Injection, Isotonic Dextrose Injection, Sterile Water Injection, Bacteriostatic Sterile Water Injection, Dextrose Lactated Ringers Injection and any combinations or mixtures thereof.

Exemplary nonaqueous parenteral vehicles include fixed oils of vegetable origin, cottonseed oil, corn oil, sesame oil, peanut oil, and combinations or mixtures thereof.

Exemplary antimicrobial agents in bacteriostatic or fungistatic concentrations include phenols, cresols, mercurials, benzyl alcohol, chlorobutanol, ethyl and propyl p-hydroxybenzoic acid esters, thimerosal, benzalkonium chloride, benzethonium chloride, and mixtures thereof.

Exemplary isotonic agents include sodium chloride, dextrose, and combinations or mixtures thereof.

Exemplary antioxidants include ascorbic acid, sodium bisulfate, and combinations or mixtures thereof.

Exemplary suspending and dispersing agents include sodium carboxymethylcelluose, hydroxypropyl methylcellulose, polyvinylpyrrolidone, any combinations or mixtures thereof.

Exemplary emulsifying agents include anionic emulsifying agents (e.g., sodium lauryl sulfate, sodium stearate, calcium oleate, and combinations or mixtures thereof), cationic emulsifying agents *(e.g.,* cetrimide), and non-ionic emulsifying agents *(e.g.,* Polysorbate 80 (Tween 80)).

Exemplary sequestering or chelating agents of metal ions include ethylenediaminetetraacetic acid (EDTA), citric acid, sorbitol, tartaric acid, phosphoric acid, and the like.

Suitable surfactants include, but are not limited to, sodium stearyl fumarate, diethanolamine cetyl sulfate, polyethylene glycol, isostearate, polyethoxylated castor oil, benzalkonium chloride, nonoxyl 10, octoxynol 9, polyoxyethylene sorbitan fatty acids (polysorbate 20, 40, 60 and 80), sodium lauryl sulfate, sorbitan esters (sorbitan monolaurate, sorbitan monooleate, sorbitan monopalmitate, sorbitan monostearate, sorbitan sesquioleate, sorbitan trioleate, sorbitan tristearate, sorbitan laurate, sorbitan oleate, sorbitan palmitate, sorbitan stearate, sorbitan dioleate, sorbitan sesqui-isostearate, sorbitan sesquistearate, sorbitan tri-isostearate), lecithin pharmaceutical acceptable salts thereof and combinations thereof. When one or more surfactants are utilized in the formulations of the invention, they may be combined, e.g., with a pharmaceutically acceptable vehicle and may be present in the final formulation, e.g., in an amount ranging from about 0.1% to about 20%, more preferably from about 0.5% to about 10%. In certain other embodiments, a surfactant can preferably be combined with one or more of the pharmaceutically acceptable vehicles previously described herein so that the surfactant or buffering agent prevents the initial stinging or burning discomfort associated with capsaicinoid administration, as a wetting agent, emulsifier, solubilizer and/or antimicrobial.

Buffering agents may also be used to provide drug stability; to control the therapeutic activity of the drug substance (Ansel, Howard C., "Introduction to Pharmaceutical Dosage Forms," 4th Ed., 1985); and/or to prevent the initial stinging or burning discomfort associated with capsaicin administration. Suitable buffers include, but are not limited to, sodium bicarbonate, sodium citrate, citric acid, sodium phosphate, pharmaceutically acceptable salts thereof, and combinations thereof. When one or more buffers are utilized in the formulations of the invention, they may be combined, e.g., with a pharmaceutically acceptable vehicle and may be present in the final formulation, e.g., in an amount ranging from about 0.1% to about 20%, more preferably from about 0.5% to about 10%. In certain embodiments, the buffer is an acetate salt, phosphate salt, citrate salt; corresponding acids of the foregoing; and combinations or mixtures thereof.

In certain embodiments, the pharmaceutical vehicle utilized to deliver the injectable capsaicinoid may comprise about 20% PEG 300, about 10 mM histidine and about 5% sucrose in water for injection. In certain other embodiments, the pharmaceutical vehicle utilized to deliver the injectable capsaicinoid may comprise about 100% PEG 300. This may be used as such or further diluted in water for injection to achieve a larger volume.

The injectable formulation may be further characterized according to the concentration of capsaicinoid in the formulation. In certain embodiments, the injectable formulation contains the capsaicinoid at a concentration ranging from about 0.01 mg/mL to about 4 mg/mL, about 0.05 mg/mL to about 3 mg/mL, about 0.1 mg/mL to about 2 mg/mL, about 0.15 mg/mL to about 2 mg/mL, about 0.2 mg/mL to about 0.8 mg/mL, about 0.25 mg/mL to about 0.6 mg/mL, about 0.25 mg/mL to about 0.5 mg/mL, about 0.3 mg/mL to about 0.5 mg/mL, about 0.3 mg/mL to about 0.4 mg/mL, about 0.35 mg/mL to about 0.45 mg/mL, or about 0.375 mg/mL to about 0.425 mg/mL. In certain preferred embodiments, the injectable formulation contains the capsaicinoid at a concentration ranging from about 0.3 mg/mL to about 4 mg/mL, about 0.05 mg/mL to about 3 mg/mL, about 0.1 mg/mL to about 2 mg/mL, about 0.15 mg/mL to about 2 mg/mL, about 0.2 mg/mL to about 0.8 mg/mL, about 0.25 mg/mL to about 0.6 mg/mL, about 0.25 mg/mL to about 0.5 mg/mL, about 0.3 mg/mL to about 0.5 mg/mL, about 0.3 mg/mL to about 0.4 mg/mL, about 0.35 mg/mL to about 0.45 mg/mL, or about 0.375 mg/mL to about 0.425 mg/mL. In certain preferred embodiments, the injectable formulation contains the capsaicinoid at a concentration ranging from about 0.3 mg/mL to about 0.4 mg/mL, or from about 0.35 mg/mL to about 0.45 mg/mL. In certain other preferred embodiments, the injectable formulation contains the capsaicinoid at a concentration ranging from about 0.3 mg/mL to about 0.4 mg/mL. The invention uses capsaicin in an amount ranging from about 0.3 mg/mL to about 0.5 mg/mL.

In certain embodiments, the aforementioned concentrations are for capsaicin. In certain embodiments, the injectable formulation contains *trans*-capsaicin at a concentration ranging from about about 0.01 mg/mL to about 4 mg/mL, about 0.05 mg/mL to about 3 mg/mL, about 0.1 mg/mL to about 2 mg/mL, about 0.15 mg/mL to about 2 mg/mL, about 0.2 mg/mL to about 0.8 mg/mL, about 0.25 mg/mL to about 0.6 mg/mL, about 0.25 mg/mL to about 0.5 mg/mL, about 0.3 mg/mL to about 0.5 mg/mL, about 0.3 mg/mL to about 0.4 mg/mL, about 0.35 mg/mL to about 0.45 mg/mL, or about 0.375 mg/mL to about 0.425 mg/mL. In certain preferred embodiments, the injectable formulation contains *trans*-capsaicin at a concentration ranging from about 0.3 mg/mL to about 0.4 mg/mL, or from about 0.35 mg/mL to about 0.45 mg/mL. In certain other preferred embodiments, the injectable formulation contains *trans*-capsaicin at a concentration ranging from about 0.3 mg/mL to about 0.5 mg/mL.

In certain embodiments, the injectable formulation contains the capsaicinoid at a concentration of about 0.1 mg/mL, 0.15 mg/mL, 0.2 mg/mL, 0.25 mg/mL, 0.3 mg/mL, 0.325 mg/mL, 0.35 mg/mL, 0.37 mg/mL, 0.38 mg/mL, 0.39 mg/mL, 0.4 mg/mL, 0.41 mg/mL, 0.42 mg/mL, 0.43 mg/mL, 0.44 mg/mL, 0.45 mg/mL, 0.475 mg/mL, 0.5 mg/mL, 0.55 mg/mL, 0.575 mg/mL, 0.6 mg/mL, 0.625 mg/mL, 0.65 mg/mL, 0.675 mg/mL, 0.7 mg/mL, 0.75 mg/mL, 0.8 mg/mL, 0.9 mg/mL, 1.0 mg/mL, 1.5 mg/mL, or 2.0 mg/mL. In certain preferred embodiments, the injectable formulation contains the capsaicinoid at a concentration of about 0.4 mg/mL. In certain other preferred embodiments, the injectable formulation contains the capsaicinoid at a concentration of about 0.325 mg/mL.

In certain embodiments, the aforementioned concentrations are for capsaicin. In certain embodiments, the injectable formulation contains trans-capsaicin at a concentration of about 0.1 mg/mL, 0.15 mg/mL, 0.2 mg/mL, 0.25 mg/mL, 0.3 mg/mL, 0.325 mg/mL, 0.35 mg/mL, 0.37 mg/mL, 0.38 mg/mL, 0.39 mg/mL, 0.4 mg/mL, 0.41 mg/mL, 0.42 mg/ mL, 0.43 mg/mL, 0.44 mg/mL, 0.45 mg/mL, 0.475 mg/mL, 0.5 mg/mL, 0.55 mg/mL, 0.575 mg/mL, 0.6 mg/mL, 0.625 mg/mL, 0.65 mg/mL, 0.675 mg/mL, 0.7 mg/mL, 0.75 mg/mL, 0.8 mg/mL, 0.9 mg/mL, 1.0 mg/mL, 1.5 mg/mL, or 2.0 mg/mL. In certain preferred embodiments, the injectable formulation contains *trans*-capsaicin at a concentration of about 0.4 mg/mL. In certain other preferred embodiments, the injectable formulation contains *trans*-capsaicin at a concentration of about 0.325 mg/mL.

The injectable formulation may be further characterized according to the solvent present to dissolve the capsaicinoid. In certain embodiments, the solvent in the injectable formulation is a mixture of water and polyethylene glycol (e.g., polyethylene glycol having a number-average molecular weight of about 300 g/mol). The relative amounts of water and polyethylene glycol in the injectable formulation may be characterized. For example, in certain embodiments, the injectable formulation contains a mixture of water and polyethylene glycol (e.g., polyethylene glycol having a number-average molecular weight of about 300 g/mol) as solvent, wherein upon a volume basis there is 3-6 times more water than polyethylene glycol. In certain embodiments, the injectable formulation contains a mixture of water and polyethylene glycol (e.g., polyethylene glycol having a number-average molecular weight of about 300 g/mol) as solvent, wherein upon a volume basis there is 4-5 times more water than polyethylene glycol. In certain embodiments, the polyethylene glycol having a number-average molecular weight in the range of about 250 g/mol to about 350 g/mol.

The injectable formulation may be further characterized according to the volume of injectable formulation administered to the acral lick granuloma or tissue proximal thereto. In certain embodiments, the volume of injectable formulation administered to the acral lick granuloma or tissue proximal thereto is in the range of about 0.1 mL to about 8 mL, about 0.2 mL to about 7 mL, about 0.3 mL to about 6 mL, about 0.4 mL to about 5 mL, about 0.5 mL to about 5 mL, about 0.6 mL to about 4 mL, about 0.7 mL to about 3 mL, about 0.8 mL to about 2.5 mL, or about 1 mL to about 2 mL. In certain other embodiments, the volume of injectable formulation administered to the acral lick granuloma or tissue proximal thereto is in the range of about 0.1 mL to about 0.5 mL, about 0.3 mL to about 0.7 mL, about 0.5 mL to about 1 mL, about 0.75 mL to about 1.5 mL, about 1 mL to about 2 mL, about 1.5 mL to about 2.5 mL, about 2.5 mL to about 3 mL, about 3.5 mL to about 4 mL, about 4.5 mL to about 5 mL, about 5 mL to about 5.5 mL, or about 5.5 mL to about 6 mL. In certain other embodiments, the volume of injectable formulation administered to the acral lick granuloma or tissue proximal thereto is in the range of about 0.3 mL to about 5 mL.

The foregoing embodiments, may be combined to describe more specific injectable formulations. For example, in certain embodiments, the injectable formulation comprises *trans*-capsaicin at a concentration ranging from about 0.3 mg/mL to about 0.4 mg/mL, water, and a polyethylene glycol (e.g., polyethylene glycol having a number-average molecular weight of 300 g/mol). In certain embodiments, the injectable formulation comprises trans-capsaicin at a concentration ranging from about 0.3 mg/mL to about 0.4 mg/mL, water, and a polyethylene glycol having a number-average molecular weight of 300 g/mol), wherein upon a volume basis there is 4-5 times more water than polyethylene glycol. In certain embodiments, the injectable formulation consists essentially of trans-capsaicin at a concentration ranging from about 0.3 mg/mL to about 0.4 mg/mL, water, and a polyethylene glycol having a number-average molecular weight of 300 g/mol, wherein upon a volume basis there is 4-5 times more water than polyethylene glycol. In certain embodiments, the injectable formulation consists of *trans-*capsaicin at a concentration ranging from about 0.3 mg/mL to about 0.4 mg/mL, water, and a polyethylene glycol having a number-average molecular weight of 300 g/mol, wherein upon a volume basis there is 4-5 times more water than polyethylene glycol.

Further description of exemplary features of formulations and components for such formulations is provided below in the section entitled "Pharmaceutical Compositions".

### Duration of Relief from Itch and/or Pain

In certain embodiments, the administering of a capsaicinoid provides relief from itch due to the acral lick granuloma for a duration of at least 2 weeks. In certain embodiments, the administering of a capsaicinoid provides relief from itch due to the acral lick granuloma for a duration of at least 4 weeks. In certain embodiments, the administering of a capsaicinoid provides relief from itch due to the acral lick granuloma for a duration of at least 6 weeks. In certain embodiments, the administering of a capsaicinoid provides relief from itch due to the acral lick granuloma for a duration of at least 8 weeks, at least 12 weeks, or at least 16 weeks.

In certain embodiments, the administering of a capsaicinoid provides relief from itch due to the acral lick granuloma for a duration of 2 weeks up to 4 months, 2 weeks up to 3 months, 2 weeks up to 2 months, 2 weeks up to 1 month, or 2 weeks up to 5 months.

In certain embodiments, the administering of a capsaicinoid provides relief from pain due to the acral lick granuloma for a duration of at least 2 weeks. In certain embodiments, the administering of a capsaicinoid provides relief from pain due to the acral lick granuloma for a duration of at least 4 weeks. In certain embodiments, the administering of a capsaicinoid provides relief from pain due to the acral lick granuloma for a duration of at least 6 weeks. In certain embodiments, the administering of a capsaicinoid provides relief from pain due to the acral lick granuloma for a duration of at least 8 weeks, or at least 12 weeks.

In certain embodiments, the administering of a capsaicinoid provides relief from pain due to the acral lick granuloma for a duration of 2 weeks up to 4 months, 2 weeks up to 3 months, 2 weeks up to 2 months, or 2 weeks up to 1 month.

### Duration of Deactivation of a Nerve Fiber

In certain embodiments, the administering of a capsaicinoid provides deactivation of a nerve fiber selected from the group consisting of a C-fiber and an A-delta fiber, each being located in or proximal to an acral lick granuloma in a canine, for a duration of at least 2 weeks. In certain embodiments, the administering of a capsaicinoid provides deactivation of a nerve fiber selected from the group consisting of a C-fiber and an A-delta fiber, each being located in or proximal to an acral lick granuloma in a canine, for a duration of at least 4 weeks. In certain embodiments, the administering of a capsaicinoid provides deactivation of a nerve fiber selected from the group consisting of a C-fiber and an A-delta fiber, each being located in or proximal to an acral lick granuloma in a canine, for a duration of at least 8 weeks, or at least 12 weeks.

In certain embodiments, the administering of a capsaicinoid provides deactivation of a nerve fiber selected from the group consisting of a C-fiber and an A-delta fiber, each being located in or proximal to an acral lick granuloma in a canine, for a duration of 2 weeks up to 4 months.

Deactivation of a nerve fiber is understood to involve rendering a primary afferent nociceptive afferent neutrally inactive or incapable of initiating or transmitting neural impulses, such as due to effects on sensory transduction or ion channels concerned with propagation of neural impulses as a result of effects at the level of the membrane either direct or indirect or degeneration of the nerve fiber and/or nerve fiber peripheral terminals.

### Characterization of Acral Lick Granuloma to Be Treated

The acral lick granuloma to be treated may be characterized according to, for example, the lateral dimensions of the lesion, the topical area of the lesion, nature of the lesion (e.g., raw tissue, open sore, and presence of bleeding), loss of hair over tissue affected by the acral lick granuloma, and presence of inflammation in tissue affected by the acral lick granuloma. Exemplary features of these characteristics are described below.

Lateral dimensions of the lesion may be characterized according to (a) the length of the long axis of the lesion and (b) the length of the long axis of the lesion. The length of the lesion along the long axis and/or the short axis may be, for example, at least about 5 mm, 10 mm, 15 mm, 20 mm, 25 mm, 30 mm, 35 mm, 40 mm, 45 mm, 50 mm, 55 mm, 60 mm, 65 mm, 70 mm, 75 mm, 80 mm, 85 mm, or 90 mm. In certain embodiments, the length of the lesion along the long axis and/or the short axis may be in the range of, for example, about 5 mm to about 30 mm, about 15 mm to about 50 mm, about 25 mm to about 60 mm, about 40 mm to about 80 mm, or about 50 mm to about 100 mm.

The lesion may be characterized according to the size of the topical area of the lesion. For example, the topical area of the lesion may be, for example, at least about 10 mm², 20 mm², 30 mm², 40 mm², 50 mm², 60 mm², 70 mm², 80 mm², 90 mm², 100 mm², 150 mm², 200 mm², 250 mm², 300 mm², 350 mm², 400 mm², 450 mm², 500 mm², 550 mm², 600 mm², 650 mm², 700 mm², 750 mm², 800 mm², 900 mm², 1000 mm², 1100 mm², 1200 mm², 1300 mm², 1400 mm², 1500 mm², 1600 mm², 1700 mm², 1800 mm², 1900 mm², or 2000 mm². In certain embodiments, the topical area of the lesion may be, for example, in the range of about 10 mm² to about 100 mm², about 100 mm² to about 250 mm², about 250 mm² to about 400 mm², about 400 mm² to about 650 mm², about 650 mm² to about 800 mm², about 800 mm² to about 1000 mm², about 1000 mm² to about 1300 mm², about 1300 mm² to about 1500 mm², about 1500 mm² to about 1700 mm², or about 1700 mm² to about 2000 mm².

The nature of the lesion due the acral lick granuloma be one or more of: raw tissue, open sore, or presence of bleeding. In certain embodiments, the lesion due the acral lick granuloma is an open sore.

The acral lick granuloma may be further characterized due to the loss of hair from tissue affected by the acral lick granuloma. In certain embodiments, there is at least a 10%, 20%, 30%, 40%, 50%, 60%, 70%, 80%, or 90% loss of hair from tissue affected by the acral lick granuloma.

The acral lick granuloma may be further characterized by the presence of inflammation in tissue affected by the acral lick granuloma. Tests reported in the literature for detecting inflammation are contemplated to be amenable here.

### Characterization of Therapeutic Benefits of Capsaicinoid Injection

Therapeutic benefits of the capsaicinoid administration may be characterized by a reduction in veterinary animal behavior indicative of discomfort at the site of the acral lick granuloma (e.g., licking the acral lick granuloma, biting at the acral lick granuloma, and scratching of the acral lick granuloma). Therapeutic benefits of the capsaicinoid administration may also be characterized by an improvement in a Client Specific Outcome Measure (CSOM), formation of a scab over a previously raw lesion, regrowing of hair over the area affected by the acral lick granuloma, reduction in the size of the acral lick granuloma, and/or reduction in the amount of inflammation in tissue affected by the acral lick granuloma. Exemplary aspects of these measures are described in more detail below.

### Reduction in Veterinary Animal Behavior Indicative of Discomfort

Therapeutic benefits of the capsaicinoid administration may be characterized by a reduction in veterinary animal behavior indicative of discomfort at the site of the acral lick granuloma (e.g., licking the acral lick granuloma, biting at the acral lick granuloma, and scratching the acral lick granuloma). The reduction in veterinary animal behavior indicative of discomfort at the site of the acral lick granuloma may be characterized according to percent reduction in the frequency of such behavior observed over a defined period of time (e.g., by comparing (i) the number of times the behavior was observed during a single day prior to receiving the capsaicinoid administration to (ii) the number of times the behavior was observed during a single day after receiving the capsaicinoid administration).

In certain embodiments, there is at least a 10%, 20%, 30%, 40%, 50%, 60%, 70%, 80%, or 90% reduction in the number of times the veterinary animal engaged in licking the acral lick granuloma on the day that is 14 days (or 28 days) after receiving the capsaicinoid compared to the average number of times the veterinary animal engaged in licking the acral lick granuloma per day on a day or period prior to receiving the capsaicinoid (e.g., for the 7 day period prior to receiving the capsaicinoid). In certain preferred embodiments, there is at least a 30%, 40%, or 50% reduction in the number of times the veterinary animal engaged in licking the acral lick granuloma on the day that is 14 days (or 28 days) after receiving the capsaicinoid compared to the average number of times the veterinary animal engaged in licking the acral lick granuloma per day on a day or period prior to receiving the capsaicinoid (e.g., for the 7 day period prior to receiving the capsaicinoid). In certain embodiments, the aforementioned reduction in the number of times the veterinary animal engaged in licking the acral lick granuloma are for when the capsaicinoid is capsaicin (e.g., capsaicin comprising greater than 95% wt/wt *trans*-capsaicin).

In certain embodiments, there is at least a 10%, 20%, 30%, 40%, 50%, 60%, 70%, 80%, or 90% reduction in the number of times the veterinary animal engaged in biting at the acral lick granuloma on the day that is 14 days (or 28 days) after receiving the capsaicinoid compared to the average number of times the veterinary animal engaged in biting at the acral lick granuloma per day on a day or period prior to receiving the capsaicinoid (e.g., for the 7 day period prior to receiving the capsaicinoid). In certain preferred embodiments, there is at least a 30%, 40%, or 50% reduction in the number of times the veterinary animal engaged in biting at the acral lick granuloma on the day that is 14 days (or 28 days) after receiving the capsaicinoid compared to the average number of times the veterinary animal engaged in biting at the acral lick granuloma per day on a day or period prior to receiving the capsaicinoid (e.g., for the 7 day period prior to receiving the capsaicinoid). In certain embodiments, the aforementioned reduction in the number of times the veterinary animal engaged in biting at the acral lick granuloma are for when the capsaicinoid is capsaicin (e.g., capsaicin comprising greater than 95% wt/wt *trans*-capsaicin).

In certain embodiments, there is at least a 10%, 20%, 30%, 40%, 50%, 60%, 70%, 80%, or 90% reduction in the number of times the veterinary animal engaged in scratching the acral lick granuloma on the day that is 14 days (or 28 days) after receiving the capsaicinoid compared to the average number of times the veterinary animal engaged in scratching the acral lick granuloma per day on a day or period prior to receiving the capsaicinoid (e.g., for the 7 day period prior to receiving the capsaicinoid). In certain preferred embodiments, there is at least a 30%, 40%, or 50% reduction in the number of times the veterinary animal engaged in scratching the acral lick granuloma on the day that is 14 days (or 28 days) after receiving the capsaicinoid compared to the average number of times the veterinary animal engaged in scratching the acral lick granuloma per day on a day or period prior to receiving the capsaicinoid (e.g., for the 7 day period prior to receiving the capsaicinoid). In certain embodiments, the aforementioned reduction in the number of times the veterinary animal engaged in scratching the acral lick granuloma are for when the capsaicinoid is capsaicin (e.g., capsaicin comprising greater than 95% wt/wt *trans*-capsaicin).

### Improvement in a Client-Specific Outcome Measure (CSOM)

Therapeutic benefits of the capsaicinoid administration may be characterized by an improvement in a Client Specific Outcome Measure (CSOM). One preferred CSOM is that described below, hereafter referred to CSOM TEST 1.

In CSOM TEST 1, up to three activities of the veterinary animal that have changed as a result of the acral lick granuloma are identified. Activities typically focus on use of the limb(s) with the acral lick granuloma lesion and on behavioral responses of the veterinary animal. Examplaryl CSOM Activities include (a) excessive licking of the lesion, (b) excessive biting at the lesion, (c) scratching at the lesion, and (d) favoring the leg with the lesion. After receiving the capsaicinoid therapy, the veterinary animals' behavior in these activities are scored relative to their state prior to the acral lick granuloma (1-no problem, 2-mildly problematic, 3-moderately problematic, 4-severely problematic, and 5-impossible). To help appropriately categorize the veterinary animals' activity impairment as either mild, moderate, or severe, the definitions in the table below are used:

| **Degree of Impairment** | **Description of Impairment** |
|---|---|
| **Mild:** far from extreme | Owner can detect impairment whereas others might not synonyms - slight, insubstantial, minor, small, weak |
| **Moderate:** not excessive or extreme | Impairment easily detected by Owner, others can observe impairment synonyms - midway, modest, medium, intermediate |
| **Severe:** intensely or extremely bad or unpleasant in degree or quality | Very obvious to any observer, condition requires evaluation or treatment synonyms - extreme, serious, highly, great, large |

A CSOM assessment should be completed prior to receiving capsaicinoid therapy (e.g., about 7 days prior to receiving capsaicinoid therapy), and then optionally again on the day of receiving capsaicinoid therapy. Thereafter, a CSOM assessment may be completed at a duration of every seven days following administration of the capsaicinoid, or every fourteen days following administration of capsaicinoid. One exemplary schedule for obtain CSOM measurements is (i) 7 days prior to receiving capsaicinoid therapy, (ii) the day of receiving capsaicinoid therapy, (iii) day 14 (±2) after receiving capsaicinoid therapy, and (iv) day 28 (±3) after receiving capsaicinoid therapy.

The reduction in CSOM score following administration of capsaicinoid may be at least a 10%, 20%, 30%, 40%, 50%, 60%, 70%, 80%, or 90% reduction at day 14 after receiving capsaicinoid therapy compared to the CSOM score observed on the day that was 7 days prior to receiving capsaicinoid therapy (or alternatively the average daily CSOM score observed over the 7 day period prior to receiving capsaicinoid therapy). In certain embodiments, the reduction in CSOM score following administration of capsaicinoid may be at least a 30% reduction at day 14 after receiving capsaicinoid therapy compared to the CSOM score observed on the day that was 7 days prior to receiving capsaicinoid therapy. In certain embodiments, reduction in CSOM score following administration of capsaicinoid may be at least a 40% reduction at day 14 after receiving capsaicinoid therapy compared to the CSOM score observed on the day that was 7 days prior to receiving capsaicinoid therapy. In certain embodiments, reduction in CSOM score following administration of capsaicinoid may be at least a 20% reduction at day 28 after receiving capsaicinoid therapy compared to the CSOM score observed on the day that was 7 days prior to receiving capsaicinoid therapy. In certain embodiments, reduction in CSOM score following administration of capsaicinoid may be at least a 30% reduction at day 28 after receiving capsaicinoid therapy compared to the CSOM score observed on the day that was 7 days prior to receiving capsaicinoid therapy. In certain embodiments, the aforementioned reduction in CSOM score is for when the capsaicinoid is capsaicin (e.g., capsaicin comprising greater than 95% wt/wt *trans*-capsaicin).

The reduction in CSOM score may also be characterized according to a range. For example, in certain embodiments, the reduction in CSOM score following administration of capsaicinoid may be in the range of about 35% to about 45%, about 30% to about 50%, about 30% to about 60%, about 30% to about 70%, about 20% to about 50%, about 20% to about 60%, about 40% to about 50%, about 40% to about 60%, about 40% to about 70%, or about 40% to about 80% reduction at day 14 after receiving capsaicinoid therapy compared to the CSOM score observed on the day that was 7 days prior to receiving capsaicinoid therapy (or alternatively the average daily CSOM score observed over the 7 day period prior to receiving capsaicinoid therapy).

In certain embodiments, reduction in CSOM score following administration of capsaicinoid may be in the range of about 20% to about 30%, about 15% to about 40%, about 10% to about 50%, about 20% to about 40%, about 20% to about 50%, about 20% to about 60%, about 20% to about 70%, about 20% to about 80%, about 30% to about 50%, about 30% to about 60%, about 30% to about 70%, about 30% to about 80%, about 40% to about 50%, about 40% to about 60%, about 40% to about 70%, or about 40% to about 80% at day 28 after receiving capsaicinoid therapy compared to the CSOM score observed on the day that was 7 days prior to receiving capsaicinoid therapy. In certain embodiments, reduction in CSOM score following administration of capsaicin may be at least a 30% reduction at day 28 after receiving capsaicinoid therapy compared to the CSOM score observed on the day that was 7 days prior to receiving capsaicinoid therapy. In certain embodiments, the aforementioned reduction in CSOM score is for when the capsaicinoid is capsaicin (e.g., capsaicin comprising greater than 95% wt/wt *trans*-capsaicin).

### Formation of a Scab Over a Previously Raw Lesion

Therapeutic benefits of the capsaicinoid administration may be characterized by formation of a scab over a previously raw lesion. The scab may form over, for example, at least about 10%, 20%, 30%, 40%, 50%, 60%, 70%, 80%, or 90% of the topical area of the lesion due to the acral lick granuloma. In certain preferred embodiments, the scab may form over at least about 30% of the topical area of the lesion due to the acral lick granuloma. In certain embodiments, the scab may form over, for example, an area that is at least about 10% to about 50%, about 20% to about 50%, about 30% to about 60%, about 40% to about 70%, about 50% to about 80%, about 60% to about 90%, about 70% to about 90%, or about 80% to about 100% of the topical area of the lesion due to the acral lick granuloma.

The extent of scab formation may be characterized at a certain point in time (e.g., 1, 2, 3, 4, 5, 6, 7, or 8 weeks after administering the capsaicinoid). In certain embodiments, the extent of scab formation may be characterized on the day that is 4 weeks after administering the capsaicinoid.

In certain embodiments, the aforementioned formation of a scab is for when the capsaicinoid is capsaicin (e.g., capsaicin comprising greater than 95% wt/wt *trans*-capsaicin).

### Regrowing of Hair Over the Area Affected by the Acral Lick Granuloma

Therapeutic benefits of the capsaicinoid administration may be characterized by regrowing of hair over the area affected by the acral lick granuloma. Hair may regrow over, for example, at least about 10%, 20%, 30%, 40%, 50%, 60%, 70%, 80%, or 90% of the topical area of the original lesion due to the acral lick granuloma. In certain preferred embodiments, hair regrows over at least about 30% of the topical area of the original lesion due to the acral lick granuloma. In certain embodiments, hair may regrow over, for example, an area that is at least about 10% to about 50%, about 20% to about 50%, about 30% to about 60%, about 40% to about 70%, about 50% to about 80%, about 60% to about 90%, about 70% to about 90%, or about 80% to about 100% of the topical area of the original lesion due to the acral lick granuloma.

The extent of regrowing of hair may be characterized at a certain point in time (e.g., 1, 2, 3, 4, 5, 6, 7, or 8 weeks after administering the capsaicinoid). In certain embodiments, the extent of regrowing of hair may be characterized on the day that is 4 weeks after administering the capsaicinoid. A more specific combination of the foregoing is where hair regrows over at least about 30% of the topical area of the original lesion due to the acral lick granuloma by day twenty-eight after administering the capsaicinoid.

In certain embodiments, the aforementioned regrowth of hair is for when the capsaicinoid is capsaicin (e.g., capsaicin comprising greater than 95% wt/wt *trans*-capsaicin).

### Reduction in Size of the Acral Lick Granuloma

Therapeutic benefits of the capsaicinoid administration may be characterized by the reduction in size of the acral lick granuloma. The reduction in size may be, for example, at least about 10%, 20%, 30%, 40%, 50%, 60%, 70%, 80%, or 90% reduction in size of the acral lick granuloma. In certain preferred embodiments, the reduction in size of the acral lick granuloma is at least about 30%. The reduction in size of the acral lick granuloma is relative to the size of acral lick granuloma on the day the veterinary animal receives the capsaicinoid (or some other day prior to receiving the capsaicinoid if so specified, such as on the day of screening performed seven days prior to receiving the capsaicinoid).

The reduction in size of the acral lick granuloma may be characterized according to the reduction in topical surface area of the acral lick granuloma at a certain day following administration of the capsaicinoid relative to the size of the acral lick granuloma on the day the capsaicinoid was first administered to the veterinary animal suffering from acral lick granuloma. Accordingly, in certain embodiments, on the fourteenth day after administration of the capsaicinoid, there is a reduction in topical surface area of the acral lick granuloma of at least about 10%, 20%, 30%, 40%, 50%, 60%, 70%, 80%, or 90% compared to the topical surface area of the acral lick granuloma on the day the capsaicinoid was administered. In certain embodiments, on the fourteenth day after administration of the capsaicinoid, there is a reduction in topical surface area of the acral lick granuloma of at least about 5% compared to the topical surface area of the acral lick granuloma on the day the capsaicinoid was administered. In certain embodiments, on the twenty-eighth day after administration of the capsaicinoid, there is a reduction in topical surface area of the acral lick granuloma of at least about 10%, 20%, 30%, 40%, 50%, 60%, 70%, 80%, or 90% compared to the topical surface area of the acral lick granuloma on the day the capsaicinoid was administered. In certain embodiments, on the twenty-eighth day after administration of the capsaicinoid, there is a reduction in topical surface area of the acral lick granuloma of at least about 5% compared to the topical surface area of the acral lick granuloma on the day the capsaicinoid was administered.

The reduction in size of the acral lick granuloma may be characterized at other time points (e.g., 1, 3, 5, 6, 7, or 8 weeks) after administering the capsaicinoid. In certain embodiments, the reduction in size of the acral lick granuloma may be characterized at 6 weeks after administering the capsaicinoid.

In certain embodiments, the aforementioned reduction in size of the acral lick granuloma is for when the capsaicinoid is capsaicin (e.g., capsaicin comprising greater than 95% wt/wt *trans-*capsaicin).

### Reduction in Amount of Inflammation in Tissue Affected by the Acral Lick Granuloma

Therapeutic benefits of the capsaicinoid administration may be characterized by a reduction in the amount of inflammation in tissue affected by the acral lick granuloma. The reduction in the amount of inflammation may be, for example, at least about 10%, 20%, 30%, 40%, 50%, 60%, 70%, 80%, or 90% reduction in the amount of inflammation. Tests reported in the literature for evaluating the amount of inflammation are contemplated to be amenable for use here. In certain preferred embodiments, the reduction in the amount of inflammation at least about 30%.

The reduction in the amount of inflammation in tissue affected by the acral lick granuloma may be characterized at a certain amount of time (e.g., 1, 2, 3, 4, 5, 6, 7, or 8 weeks) after administering the capsaicinoid. In certain embodiments, the reduction in the amount of inflammation in tissue affected by the acral lick granuloma may be characterized at 2 weeks or 4 weeks after administering the capsaicinoid.

In certain embodiments, the aforementioned reduction in the amount of inflammation in tissue affected by the acral lick granuloma is for when the capsaicinoid is capsaicin (e.g., capsaicin comprising greater than 95% wt/wt *trans*-capsaicin).

### Anesthetic

To help control pain that may be experienced upon initial administration of the capsaicinoid, an anesthetic agent may be administered to the veterinary animal (e.g., a canine) prior to administration of the capsaicinoid.

In certain embodiments, the anesthetic agent is an agent that causes general sedation. For example, in certain embodiments, general anesthesia is administered (e.g., to the canine) prior to administering the capsaicinoid. Exemplary general anesthetics include, for example, propofol, isoflurane, sevoflurane, and desflurane.

In certain embodiments, a local anesthetic may be administered prior to or concurrently with said dose of capsaicinoid in an amount and location effective to attenuate an initial hyperalgesic effect of the administered dose of capsaicinoid. The local anesthetic may be administered, e.g., by direct injection into the site where said dose of capsaicinoid is administered, or as a proximal, regional, somatic, or neuraxial block.

In certain embodiments, the local anesthetic is a caine analgesic. Exemplary caine analgesics include, for example, lidocaine, dibucaine, bupivacaine, ropivacaine, etidocaine, tetracaine, procaine, chlorocaine, prilocaine, mepivacaine, xylocaine, 2-chloroprocaine, and pharmaceutically acceptable salts thereof.

In certain embodiments, the anesthetic agent is a caine analgesic administered proximal to the granuloma. In certain embodiments, the caine analgesic is lidocaine or a pharmaceutically acceptable salt thereof.

### II. PHARMACEUTICAL COMPOSITIONS

Pharmaceutical compositions are described comprising a capsaicinoid. In certain embodiments, the pharmaceutical compositions preferably comprise a therapeutically-effective amount of one or more of the capsaicinoids described above, formulated together with one or more pharmaceutically acceptable carriers.

As described in detail below, the pharmaceutical compositions described may be specially formulated for administration for parenteral administration by, for example, subcutaneous injection as, for example, a sterile solution.

Liquid dosage forms for administration of the compounds include pharmaceutically acceptable emulsions, microemulsions, solutions, and suspensions. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Pharmaceutical compositions suitable for parenteral administration may comprise one or more compounds d in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Exemplary suitable aqueous and nonaqueous carriers which may be employed in the pharmaceutical compositions include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the subject compounds may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

When the compounds described are administered as pharmaceuticals, to veterinary animals, they can be given as a pharmaceutical composition containing, for example, 0.01 to 99% (more preferably, 0.01 to 0.5%) of active ingredient in combination with a pharmaceutically acceptable carrier. Unit dose injectable liquid volumes can be up to, in certain embodiments, 10 mL.

Actual dosage levels of the active ingredients in the pharmaceutical compositions may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular described compound employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the canine being treated, and like factors well known in the medical arts.

A veterinarian having ordinary skill in the art can readily determine and prescribe the effective amount of the pharmaceutical composition required. For example, the veterinarian could start doses of the described compounds employed in the pharmaceutical composition at levels lower than that required in order to achieve the desired therapeutic effect and gradually increase the dosage until the desired effect is achieved.

In general, a suitable dose of a described compound will be that amount of the compound which is the lowest dose effective to produce a therapeutic effect. Such an effective dose will generally depend upon the factors described above. Preferably, the compounds are administered at about 0. 1 mg to about 10 mg, more preferably at about 0.1 mg to about 4 mg, even more preferably at about 0.2 mg to about 2 mg. When the compounds described herein are co-administered with another agent *(e.g.,* as sensitizing agents), the effective amount may be less than when the agent is used alone.

If desired, the effective dose of the active compound may be administered as two, three, four, five, six or more sub-doses administered separately at appropriate locations, optionally, in unit dosage forms to achieve saturation of the appropriate tissues with the active compound.

The description above describes multiple aspects and embodiments of the invention. The patent application specifically contemplates all combinations and permutations of the aspects and embodiments.

### III. DEFINITIONS

To facilitate an understanding of the present invention, a number of terms and phrases are defined below.

The terms "a" and "an" as used herein mean "one or more" and include the plural unless the context is inappropriate.

The phrase "tissue proximal to the granuloma" refers to tissue that is located within about 1 cm of the granuloma. In certain embodiments, the tissue is located within about 0.8, 0.6, 0.4, 0.2, or 0.1 cm of the granuloma.

The present invention encompasses the various geometric isomers and mixtures thereof resulting from the arrangement of substituents around a carbon-carbon double bond or arrangement of substituents around a carbocyclic ring. Substituents around a carbon-carbon double bond are designated as being in the *"Z"* or *"E"* configuration wherein the terms *"Z"* and *"E"* are used in accordance with IUPAC standards. Unless otherwise specified, structures depicting double bonds encompass both the *"E"* and *"Z"* isomers.

Substituents around a carbon-carbon double bond alternatively can be referred to as "cis" or "trans," where "cis" represents substituents on the same side of the double bond and "trans" represents substituents on opposite sides of the double bond. Mixtures of compounds wherein the substituents are disposed on both the same and opposite sides of plane of the ring are designated "cis/trans."

As used herein, the terms "subject" and "patient" refer to organisms to be treated by the methods described, such as canines. Veterinary animals include canine, felines, equines, and the like.

As used herein, the term "effective amount" refers to the amount of a compound (*e.g*., a compound described) sufficient to effect beneficial or desired results. An effective amount can be administered in one or more administrations, applications or dosages and is not intended to be limited to a particular formulation or administration route. As used herein, the term "treating" includes any effect, *e.g*., lessening, reducing, modulating, ameliorating or eliminating, that results in the improvement of the condition, disease, disorder, and the like, or ameliorating a symptom thereof.

As used herein, the term "pharmaceutical composition" refers to the combination of an active agent with a carrier, inert or active, making the composition especially suitable for diagnostic or therapeutic use *in vivo* or *ex vivo.*

As used herein, the term "pharmaceutically acceptable carrier" refers to any of the standard pharmaceutical carriers, such as a phosphate buffered saline solution, water, emulsions *(e.g.,* such as an oil/water or water/oil emulsions), and various types of wetting agents. The compositions also can include stabilizers and preservatives. For examples of carriers, stabilizers and adjuvants, *see e.g.,* Martin, Remington's Pharmaceutical Sciences, 15th Ed., Mack Publ. Co., Easton, PA [1975].

As used herein, the term "pharmaceutically acceptable salt" refers to any pharmaceutically acceptable salt *(e.g.,* acid or base) of a described compound which, upon administration to a subject, is capable of providing a compound described or an active metabolite or residue thereof. As is known to those of skill in the art, "salts" of the compounds described may be derived from inorganic or organic acids and bases. Exemplary acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds and their pharmaceutically acceptable acid addition salts.

Exemplary bases include, but are not limited to, alkali metal (*e.g*., sodium) hydroxides, alkaline earth metal (*e.g*., magnesium) hydroxides, ammonia, and compounds of formula NW₄⁺, wherein W is C₁₋₄ alkyl, and the like.

Exemplary salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds described compounded with a suitable cation such as Na⁺, NH₄⁺, and NW₄⁺ (wherein W is a C₁₋₄ alkyl group), and the like.

For therapeutic use, salts of the compounds described are contemplated as being pharmaceutically acceptable. However, salts of acids and bases that are non-pharmaceutically acceptable may also find use, for example, in the preparation or purification of a pharmaceutically acceptable compound.

Throughout the description, where compositions are described as having, including, or comprising specific components, or where processes and methods are described as having, including, or comprising specific steps, it is contemplated that, additionally, there are described compositions that consist essentially of, or consist of, the recited components, and that there are processes and methods described that consist essentially of, or consist of, the recited processing steps.

As a general matter, compositions specifying a percentage are by weight unless otherwise specified. Further, if a variable is not accompanied by a definition, then the previous definition of the variable controls.

### EXAMPLES

The invention now being generally described, will be more readily understood by reference to the following example, which is included merely for purposes of illustration of certain aspects and embodiments of the present invention, and is not intended to limit the invention.

### Example 1 - Capsaicin Injection for Treatment of Canines Suffering from Acral Lick Granuloma

A clinical study was performed to evaluate the effect of injectable capsaicin in treating acral lick granuloma in canines. A total of six dogs suffering from acral lick granuloma were treated using injectable capsaicin and evaluated. Experimental procedures are described in Part I below. Results are provided in Part II below.

### Part I: Experimental Procedures

Six dogs suffering from acral lick granuloma were treated using injectable capsaicin and evaluated. Each dog satisfied the inclusion criteria set forth in Table 1 below. Additionally, each dog did not have any of the exclusion criteria set forth in Table 2 below.

**Table 1. Inclusion Criteria**

| **Criteria No.** | **Description of the Criteria** |
|---|---|
| 1. | Client-owned dog with excessive licking of a lesion consistent with a diagnosis of acral lick granuloma. The lesion can initially be red, shiny, swollen, hairless, irritated, and bleeding, similar to a hot spot (wet eczema). The dog's incessant licking of the lesion eventually results in a raised, thickened, hard, firm, oval plaque that is insensitive to pressure. |
| 2. | In generally good health or stabilized for chronic conditions that, in the opinion of the veterinarian, will not interfere with study evaluations and will not prevent the dog from completing the study. |
| 3. | Owner willing and able to complete the study procedures and pain scales, and to communicate meaningfully with the study personnel. |
| 4. | Owner willing and able to sign an Informed Consent Form (ICF). |

**Table 2. Exclusion Criteria**

| **Criteria No.** | **Description of the Criteria** |
|---|---|
| 1. | Dogs intended for breeding; pregnant or lactating females. |
| 2. | Clinical pathology findings considered abnormal and clinically significant, apart from previously identified stable chronic conditions. |
| 3. | Evidence of dermatitis with severe bacterial infection associated with the lesion (mild-moderate, superficial bacterial infection is allowed). |
| 4. | Surgery or other invasive procedures planned for the lesion within the study period. |
| 5. | Concurrent disease that would affect study assessments of performance, function and pain and which may include: infectious (Lyme disease, pyoderma), neoplasia, autoimmune disorders, neurological/neuromuscular disorders, etc. |
| 6. | Use of topical treatments and alternative therapies for acral lick granuloma within the specified washout periods prior to Day 0 (See Concomitant Medications section.). |
| 7. | Dog lifestyle management changes (e.g., significant weight loss, dietary changes, and changes in level of exercise) must not be undertaken within two weeks prior to Day 0 until Day 90. |
| 8. | Any dog that is fractious or uncooperative to the extent that this may interfere with study evaluations. |

The breed and gender of dogs enrolled in the study is provided in Table 3 below.

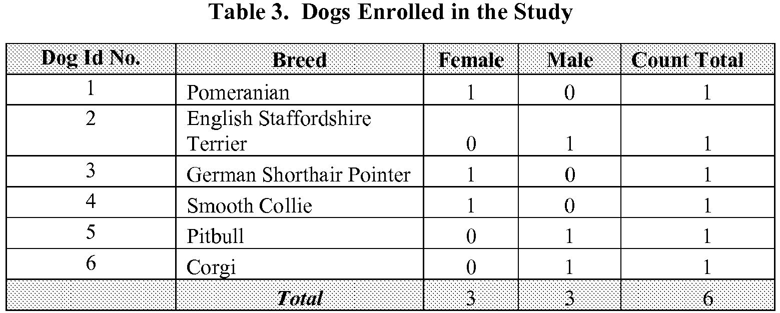

Lesions observed on each dog in the study due to acral lick granuloma are described in Table 4 below.

**Table 4. Lesions Observed on Dogs Enrolled in the Study**

| **Dog Ia No.** | **Breed** | **Lesions** |
|---|---|---|
| 1 | Pomeranian | Two lesions. 1) Left front dorsal carpus. 50 mm x 4.5 mm. 2) Left front dorsal metacarpus. 40 mm x 32 mm |
| 2 | English Staffordshire Terrier | Two lesions. 1) Right front distal dorsal carpus. 10 mm x 10 mm. 2) Right front proximal carpus. 10 mm x 7 mm |
| 3 | German Shorthair Pointer | One lesion. 1) Left lateral carpus. 13 mm x 10 mm |
| 4 | Smooth Collie | Two lesions. 1) Left front lateral mid-radius. 5 mm x 5 mm. 2) Left hind medial distal metatarsus. 15 mm x 7 mm |
| 5 | Pitbull | Five lesions. 1) Dorsal right carpus. 58.4 mm x 22.5 mm. 2) Dorsal medial left carpus. 44.6 mm x 15.2 mm. 3) Dorsal lateral left carpus. 23.7 mm x 21.7 mm. 4) Dorsal left tarsus. 39.5 mm x 19.3 mm. 5) Dorsal distal left metatarsus. 23.5 mm x 15.0 mm |
| 6 | Corgi | One lesion. 1) Right dorsal carpus. 26.4 mm x 16.4 mm |

Dogs were screened and selected for the study based on the presence of a lesion typical of acral lick granuloma and behavior identifiable as excessive licking of the lesion. In cases of more than one lesion, the more severely affected lesions were identified and documented. In the event that multiple lesions were identified, the first lesion listed was determined to be the index lesion.

At a Screening visit, the index lesion was examined. The diagnosis of the index granuloma was made based on characteristics of the lesion and history of licking. Veterinary personnel reviewed with each dog owner the concept and implementation of an Owner-derived efficacy assessment, the Client Specific Outcome Measure (CSOM, based loosely on the CSOM developed for use with osteoarthritis (Gingerich D, and Strobel J. in "Use of client-specific outcome measures to assess treatment effects in geriatric, arthritic dogs: Controlled clinical evaluation of a nutraceutical," Vet. Ther. (2003) vol. 4, pages 56-66)), to assess the severity and progression of licking behavior associated with the acral lick granuloma. Up to three activities were identified and scored relative to their state prior to the acral lick granuloma (1-no problem, 2-mildly problematic, 3-moderately problematic, 4-severely problematic, 5-impossible). The CSOM assessment was completed at Screening Day (i.e., seven days before administration of capsaicin, also referred to as Day -7), Day 0, Day 14 (±2), and Day 28 (±3) until study termination.

After the screening visit, the dog returned to the site on Day 0 for injection with Study Drug. As a general procedure, dogs were anesthetized using a short-acting anesthetic protocol before injection of the Study Drug. If propofol was used, it was used for induction prior to gas anesthesia but not as a drip to maintain anesthesia. The area to be injected with Study Drug was prepared using site disinfection procedures that are routinely used at veterinary hospital.

Veterinary personnel followed up with the dog owner twenty-four (24) hours after Day 0 to ensure the injection procedure was well tolerated by the dog. Dogs returned to the clinic for follow-up examinations on Day 14 and 28 of the study.

The Study Drug was trans-capsaicin in a solution of polyethylene glycol 300 (PEG 300) and water for injection. The Study Drug was prepared by diluting (i) a solution of PEG 300 containing trans-capsaicin at a concentration of 2 mg/mL (hereinafter "Solution A") with (ii) sterile water for injection. Solution A was diluted with a volume of sterile water for injection (WFI) in an amount equal to 4 to 5-fold to produce the Study Drug.

Study Drug was administered to the dog by subcutaneous injection at several locations under the index acral lick granuloma. The goal of the injection was to infiltrate the Study Drug at the junction between the granuloma and healthy tissue throughout the area of the lesion. The dose of *trans*-capsaicin per lesion to be administered was approximately 1.5 µg of *trans*-capsaicin per mm² of lesion. Each lesion to be injected was measured (mm) for long axis (length) and short axis (width). The approximate surface area was calculated based on an elliptical shape. Based on the calculated lesion areas, the calculated dose of *trans*-capsaicin per lesion ranged from <0.10 mg to 2.65 mg of *trans*-capsaicin. Dog reactions during and immediately after the injection procedure were recorded in the study records. Injections of Study Drug were performed using a 22 gauge needle.

Use of the following medications by any route of administration were not started during the study. At the time of the Screening Visit, if the dog was currently on a stable regimen that was expected to continue through the study, the medication was allowed to continue as long as the medications were documented.
- NSAIDs, unless already in use for an unrelated condition (e.g., osteoarthritis)
- Analgesics
- Short-acting systemic corticosteroids
- Mid- to long-acting systemic corticosteroids
- Topical corticosteroids

As noted above, the severity of licking behavior caused by the acral lick granuloma was measured by the owner using a modification of a previously published system (Gingerich and Strobel 2003 noted above). During Screening, dog owners were interviewed by veterinary personnel trained in CSOM assessments (e.g., Study Coordinator) to identify one to three activities that have changed as a result of the acral lick granuloma. Activities focused on the use of the limb(s) with the index lesion and on behavioral responses. For example, excessive licking of the granuloma would be a behavioral response. For dogs with multiple acral lick granulomas, activities were carefully selected to ensure that any treatment effect was easily observed for the treated lesion. Activities that were significantly affected by the untreated lesions should be avoided.

The Owner was asked to give examples as prompts, and were encouraged to customize them for their dog and their situation. Example CSOM Activities that were chosen include: (a) excessive licking of the lesion, (b) excessive biting at the lesion, (c) scratching at the lesion, and (d) favoring the leg with the lesion.

Owners choose an activity at Screening and discussed with the study staff the appropriateness of the activity for the duration of the observation period. After the administration of Study Drug, owners were asked to specifically indicate both places and times when they observe these activities impaired, e.g., "licking the lesion last thing at night", or "biting at the lesion after it is touched or bumped."

The CSOM score was measured during the Screening visit with assistance from the appropriate veterinary personnel. The Owner is asked by the study staff responsible for measuring Owner-derived efficacy assessments to rate the degree of impairment:
- 1-no problem,
- 2-mildly problematic
- 3-moderately problematic
- 4-severely problematic
- 5-impossible

These ratings described the chosen observed activities compared to when the dog was considered normal (e.g., a specific age) or if the dog was obtained by the owner already impaired, compared to what the owner would consider normal for a dog of that same age and breed. To help owners appropriately categorize their dog's activity impairment as either mild, moderate, or severe, the owners were provided definitions and general descriptions of each term as well as other similar words that might be used to describe that level of impairment. These are described in Table 5 below.

**Table 5.**

| **Degree of Impairment** | **Description of Impairment** |
|---|---|
| **Mild:** far from extreme | Owner can detect impairment whereas others might not synonyms - slight, insubstantial, minor, small, weak |
| **Moderate:** not excessive or extreme | Impairment easily detected by Owner, others can observe impairment synonyms - midway, modest, medium, intermediate |
| **Severe:** intensely or extremely bad or unpleasant in degree or quality | Very obvious to any observer, condition requires evaluation or treatment synonyms - extreme, serious, highly, great, large |

Physical examinations of the dog including the injection site observation were conducted. General and dermatologic exams, including a description, measurements (dimensions), and digital photograph of the index lesion were collected.

### Part II: Results

The experimental procedure described above produced an average reduction in CSOM of 41% at Day 14 and an average reduction in CSOM of 26% at Day 28 compared with baseline. The CSOM results indicate that the trans-capsaicin injection provided a benefit to the dogs suffering from acral lick granuloma.

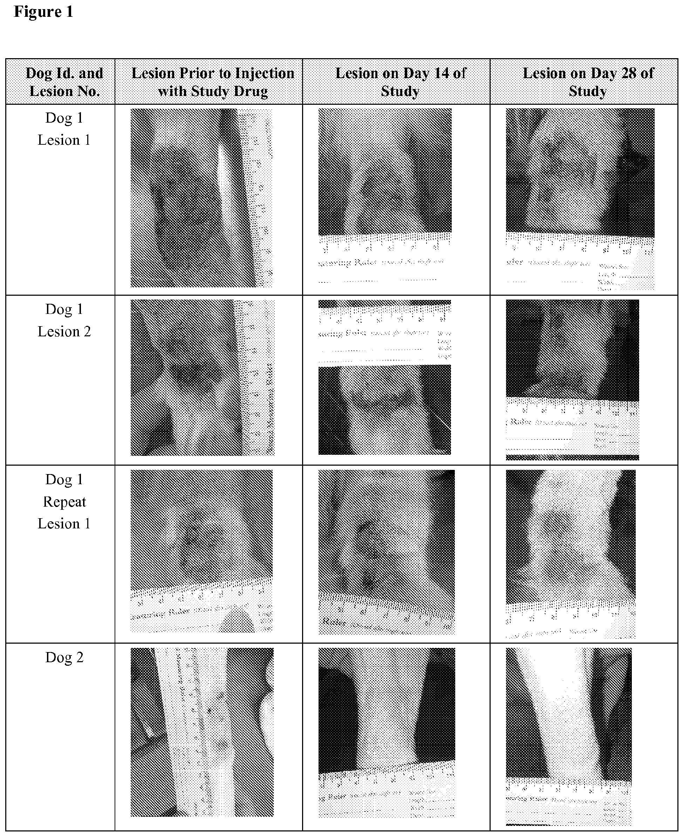

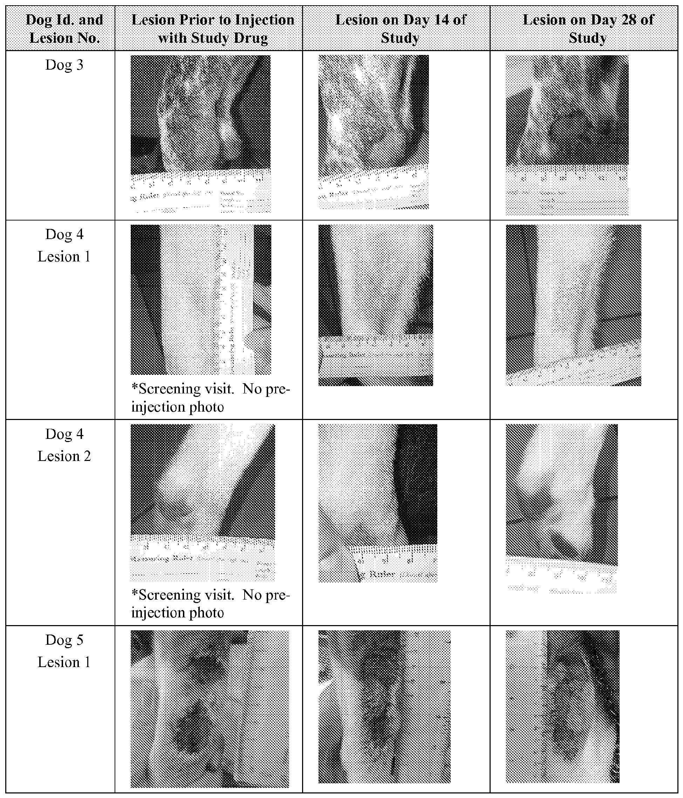

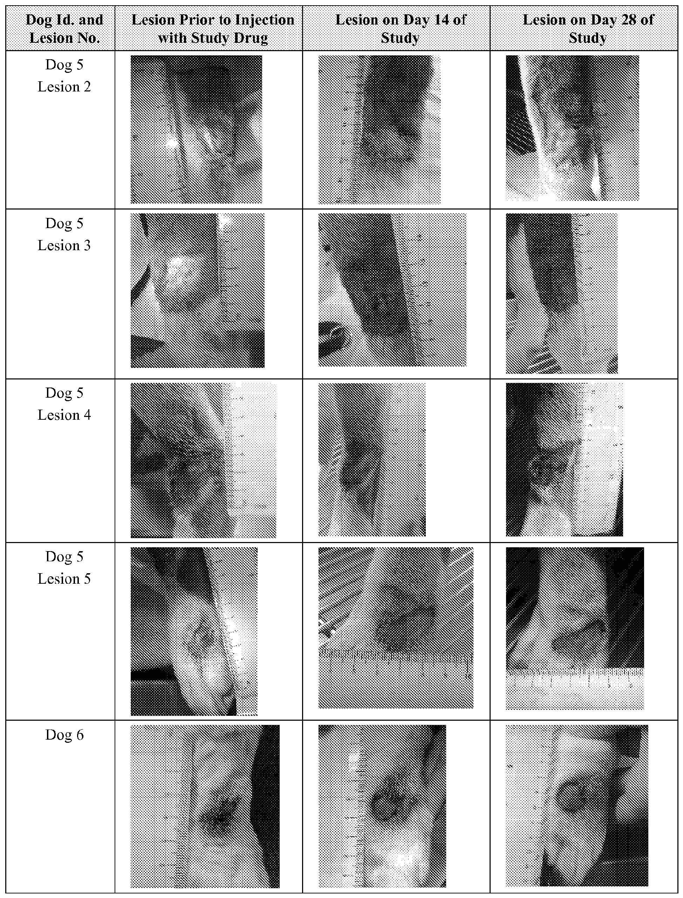

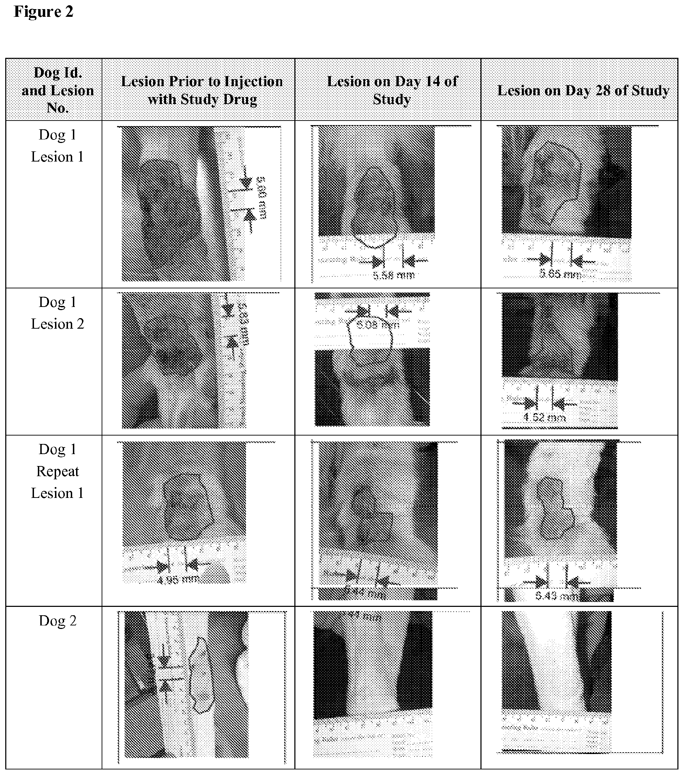

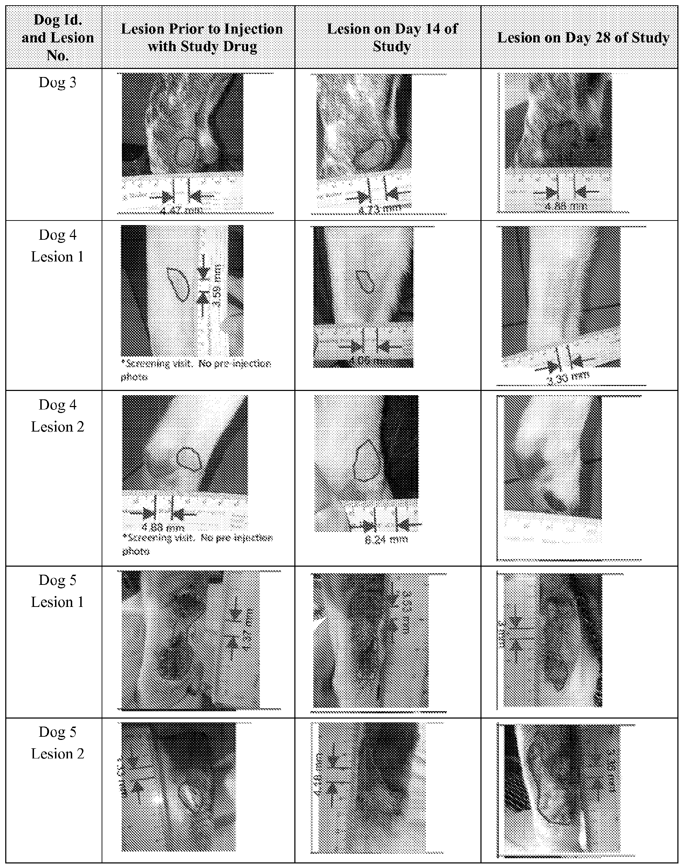

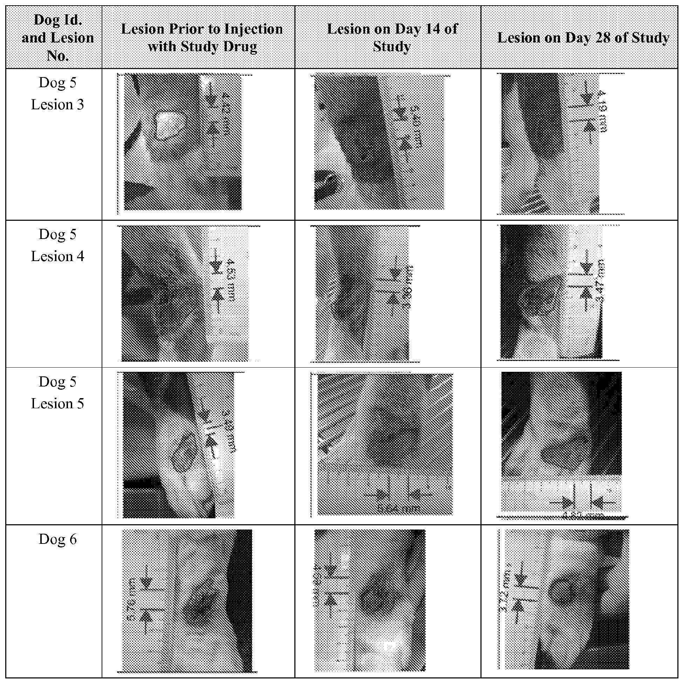

CSOM values observed during the study are provided in Table 6 below. Characterization of the size of the lesions in the dogs is provided in Tables 7-9 below. An illustration of the lesion(s) from each dog prior to injection with Study Drug are provided in Figure 1, along with illustrations of the lesions from each dog at Day 14 and separately at Day 28 of the study. Figure 2 provides illustrations of the lesions from each dog prior to injection of Study Drug, at Day 14 of the study, and at Day 28 of the study, where the lesion is traced for Adobe Acrobat IX determination of lesion area.

**Table 6. CSOM Values (sum of identified activities)**

| **Dog Id. No.** | **CSOM Baseline** | **CSOM Day 14** | **CSOM Day 28** | **Delta Day 14- baseline** | **Delta Day 28-baseline** | **Percent Change to Day 14** | **Percent Change to Day 28** |
|---|---|---|---|---|---|---|---|
| 1 | 12 | 6 | 8 | -6 | -4 | -50% | -33% |
| 2 | 6 | 2 | 2 | -4 | -4 | -67% | -67% |
| 3 | 8 | 4 | 4 | -4 | -4 | -50% | -50% |
| 4 | 2 | 1 | 1 | -1 | -1 | -50% | -50% |
| 5 | 9 | 7 | 11 | -2 | 2 | -22% | 22% |
| 6 | 10 | 9 | 12 | -1 | 2 | -10% | 20% |
| **Average** | **7.8** | **4.8** | **6.3** | **-3.0** | **-1.5** | **-41%** | **-26%** |

**Table 7. Long Axis and Short Axis of Lesion**

| **Identification** | | **Lesion size (mm)** | | | | | |
|---|---|---|---|---|---|---|---|
| Dog Id. No. | Lesian No. | pre-dose long axis | pre-dose short axis | Day 14-long axis | Day 14-short axis | Day 28-long axis | Day 28-short axis |
| 1 | 1 | 50 | 45 | 55 | 47 | 55 | 50 |
| 1 | 2 | 40 | 32 | 45 | 30 | 45 | 30 |
| 2 | 1 | 10 | 10 | 7 | 5 | 10 | 5 |
| 2 | 2 | 10 | 7 | 0 | 0 | 5 | 3 |
| 3 | 1 | 13 | 10 | 25 | 20 | 20 | 15 |
| 4 | 1 | 5 | 5 | 5 | 5 | 0 | 0 |
| 4 | 2 | 15 | 7 | 5 | 5 | 0 | 0 |
| 5 | 1 | 58.4 | 22.5 | 65.3 | 23.3 | 88.2 | 25.5 |
| 5 | 2 | 44.6 | 15.2 | 46.5 | 23.6 | 65.2 | 34.8 |
| 5 | 3 | 23.7 | 21.7 | 18.2 | 15.1 | 23.3 | 18 |
| 5 | 4 | 39.5 | 19.3 | 39.7 | 15.5 | 46.1 | 20.7 |
| 5 | 5 | 23.5 | 15 | 29.7 | 14.3 | 31.1 | 17.4 |
| 6 | 1 | 26.4 | 16.4 | 25.3 | 14.7 | 20.5 | 20.7 |

**Table 8. Lesion Surface Area Values (based on approximation of an ellipse)**

| **Dog Id. No.** | **Lesion No.** | **Screening sq mm** | **Day 14 sq mm** | **Day 28 sq mm** | **Delta Day 14-baseline** | **Delta Day 28-baseline** | **Percent Change to Day 14** | **Percent Change to Day 28** |
|---|---|---|---|---|---|---|---|---|
| 1 | 1 | 1767 | 2030 | 2160 | 263 | 393 | 15% | 22% |
| 1 | 2 | 1005 | 1060 | 1060 | 55 | 55 | 5% | 5% |
| 2 | 1 | 79 | 27 | 39 | -51 | -39 | -65% | -50% |
| 2 | 2 | 55 | 0 | 12 | -55 | -43 | -100% | -79% |
| 3 | 1 | 102 | 393 | 236 | 291 | 134 | 285% | 131% |
| 4 | 1 | 20 | 20 | 0 | 0 | -20 | 0% | -100% |
| 4 | 2 | 82 | 20 | 0 | -63 | -82 | -76% | -100% |
| 5 | 1 | 1032 | 1195 | 1766 | 163 | 734 | 16% | 71% |
| 5 | 2 | 532 | 862 | 1782 | 329 | 1250 | 62% | 235% |
| 5 | 3 | 404 | 216 | 329 | -188 | -75 | -47% | -18% |
| 5 | 4 | 599 | 483 | 749 | -115 | 151 | -19% | 25% |
| 5 | 5 | 277 | 334 | 425 | 57 | 148 | 20% | 54% |
| 6 | 1 | 340 | 292 | 333 | -48 | -7 | -14% | -2% |
| **Average** | | **484** | **533** | **684** | **49** | **200** | **6%** | **15%** |

**Table 9. Lesion Surface Area Values (based on Adobe Acrobat XI measurement tool)**

| **Dog Id. No.** | **Lesion No.** | **Total Size of Lesion at Screening sq mm** | **Total Size of Lesion at Day 14 sq mm** | **Total Size of Lesion at Day 28 sq mm** | **Delta Day 14-baseline** | **Delta Day 28-baseline** | **Percent Change Day 14** | **Percent Change Day 28** |
|---|---|---|---|---|---|---|---|---|
| 1 | 1 | 1207 | 704 | 766 | -503 | -441 | -42% | -37% |
| 1 | 2 | 571 | 568 | 509 | -4 | -62 | -1% | -11% |
| 2 | 1 | 924 | 0 | 0 | -924 | -924 | -100% | -100% |
| 2 | 2 | | | | | | | |
| 3 | 1 | 195 | 232 | 271 | 37 | 76 | 19% | 39% |
| 4 | 1 | 260 | 114 | 0 | -146 | -260 | -56% | -100% |
| 4 | 2 | 136 | 161 | 0 | 25 | -136 | 18% | -100% |
| 5 | 1 | 969 | 1252 | 1564 | 282 | 594 | 29% | 61% |
| 5 | 2 | 370 | 660 | 1432 | 290 | 1062 | 78% | 287% |
| 5 | 3 | 316 | 86 | 100 | -230 | -216 | -73% | -68% |
| 5 | 4 | 473 | 579 | 720 | 106 | 247 | 22% | 52% |
| 5 | 5 | 405 | 303 | 358 | -103 | -48 | -25% | -12% |
| 6 | 1 | 209 | 290 | 355 | 81 | 146 | 39% | 70% |
| **Average** | | **503** | **462** | **557** | **-91** | **3** | **-8%** | **7%** |

### EQUIVALENTS

Scope of the invention is indicated by the appended claims.

## Claims

1. A liquid capsaicin injectable formulation for use in treating acral lick granuloma in a canine, wherein the liquid capsaicin injectable formulation is for administering to the canine by multiple subcutaneous injections to tissue proximal to the lower boundary of the granuloma, wherein, after the first subcutaneous injection of the formulation to tissue proximal to the lower boundary of the granuloma, any subsequent subcutaneous injection of the formulation into tissue proximal to the lower boundary of the granuloma is performed at a location laterally distal to any prior injection of the formulation to tissue proximal to the lower boundary of the granuloma, to thereby disperse capsaicin throughout the area under the acral lick granuloma in proximity to the boundary between healthy tissue and the granuloma; wherein
the administering delivers capsaicin in an amount ranging from about 1.2 µg to about 1.8 µg of capsaicin per square millimeter of topical surface area of the acral lick granuloma;
the liquid capsaicin injectable formulation contains capsaicin in an amount ranging from about 0.3 mg/mL to about 0.5 mg/mL; and
the capsaicin comprises greater than about 95% trans-capsaicin.

2. The formulation for use according to claim 1, wherein the use delivers capsaicin in an amount of about 1.5 µg of capsaicin per square millimeter of topical surface area of the acral lick granuloma.

3. The formulation for use according to claim 1 or 2, wherein the multiple subcutaneous injections of the liquid capsaicin injectable formulation to tissue proximal to the lower boundary of the granuloma are injections of the liquid capsaicin injectable formulation into healthy tissue proximal to the lower boundary of the granuloma.

4. The formulation for use according to claim 3, wherein the use further comprises injecting a liquid capsaicin injectable formulation into acral lick granuloma tissue.

5. The formulation for use according to any one of claims 1-3, wherein the use delivers from about 0.3 mL to about 5 mL of the formulation.

6. The formulation for use according to any one of claims 1-5, wherein the use provides one or more of the following:
• at least a 30% reduction in the number of times the canine engaged in licking the acral lick granuloma on the day that is 28 days after receiving the capsaicin compared to the average number of times the canine engaged in licking the acral lick granuloma per day for the 7 day period prior to receiving the capsaicin;
• at least a 30% reduction in the number of times the canine engaged in biting at the acral lick granuloma on the day that is 28 days after receiving the capsaicin compared to the average number of times the canine engaged in biting at the acral lick granuloma per day for the 7 day period prior to receiving the capsaicin;
• at least a 30% reduction in the number of times the canine engaged in scratching the acral lick granuloma on the day that is 28 days after receiving the capsaicin compared to the average number of times the canine engaged in scratching the acral lick granuloma per day for the 7 day period prior to receiving the capsaicin; or
• hair regrows over at least about 30% of the topical area of the original lesion due to the acral lick granuloma by day twenty-eight after receiving the capsaicin.

7. The formulation for use according to any one of claims 1-6, wherein the administering provides one or more of the following:
• at least a 40% reduction in a client-specific outcome measure (CSOM) score at day 14 after receiving capsaicin compared to the CSOM score observed on the day that was 7 days prior to receiving capsaicin; or
• at least a 20% reduction in CSOM score at day 28 after receiving capsaicin compared to the CSOM score observed on the day that was 7 days prior to receiving capsaicin.

8. The formulation for use according to any one of claims 1-7, further **characterized by** providing relief from itch due to the acral lick granuloma for a duration of at least 6 weeks.

9. The formulation for use according to of any one of claims 1-8, further **characterized by** providing relief from pain due to the acral lick granuloma for a duration of at least 6 weeks.

10. The formulation for use according to any one of claims 1-9, further **characterized by** providing deactivation of a nerve fiber selected from the group consisting of a C-fiber and an A-delta fiber, each being located in or proximal to an acral lick granuloma in the canine, for a duration of at least 4 weeks.

11. The formulation for use according to any one of claims 1-10, wherein the use comprises administering, prior to the capsaicinoid, an anesthetic agent.

12. The formulation for use according to claim 11, wherein the anesthetic agent is an agent that causes general sedation of the canine.

13. The formulation for use according to claim 11, wherein the anesthetic agent is a canine analgesic administered proximal to the granuloma.

14. The formulation for use according to claim 13, wherein the canine analgesic is lidocaine or a pharmaceutically acceptable salt thereof.

## Patentansprüche

1. Injizierbare flüssige Capsaicin-Formulierung zur Verwendung bei der Behandlung eines Akral-Lick-Granuloms bei einem Hund, wobei die injizierbare flüssige Capsaicin-Formulierung zur Verabreichung an den Hund durch mehrfache subkutane Injektionen in Gewebe proximal der unteren Grenze des Granuloms dient, wobei nach der ersten subkutanen Injektion von der Formulierung in Gewebe proximal der unteren Grenze des Granuloms jede nachfolgende subkutane Injektion der Formulierung in Gewebe proximal der unteren Grenze des Granuloms an einer Stelle durchgeführt wird, die seitlich distal zu einer vorherigen Injektion der Formulierung in Gewebe proximal der unteren Grenze des Granuloms liegt, um dadurch Capsaicin im gesamten Bereich unter dem Akral-Lick-Granulom in der Nähe der Grenze zwischen gesundem Gewebe und Granulom zu verteilen; wobei
die Verabreichung Capsaicin in einer Menge im Bereich von etwa 1,2 µg bis etwa 1,8 µg Capsaicin pro Quadratmillimeter topischer Oberfläche des Akral-Lick-Granuloms liefert;
die flüssige injizierbare Capsaicin-Formulierung Capsaicin in einer Menge im Bereich von etwa 0,3 mg/ml bis etwa 0,5 mg/ml enthält, und
das Capsaicin mehr als etwa 95 % trans-Capsaicin umfasst.

2. Formulierung zur Verwendung nach Anspruch 1, wobei die Verwendung Capsaicin in einer Menge von etwa 1,5 µg Capsaicin pro Quadratmillimeter topischer Oberfläche des Akral-Lick-Granuloms liefert.

3. Formulierung zur Verwendung nach Anspruch 1 oder 2, wobei die mehrfachen subkutanen Injektionen der flüssigen Capsaicin-injizierbaren Formulierung in Gewebe proximal der unteren Grenze des Granuloms Injektionen der flüssigen Capsaicin-injizierbaren Formulierung in gesundes Gewebe proximal der unteren Grenze des Granuloms sind.

4. Formulierung zur Verwendung nach Anspruch 3, wobei die Verwendung ferner das Injizieren einer flüssigen Capsaicin-injizierbaren Formulierung in Akral-Lick-Granulomgewebe umfasst.

5. Formulierung zur Verwendung nach einem der Ansprüche 1 bis 3, wobei die Verwendung etwa 0,3 ml bis etwa 5 ml der Formulierung liefert.

6. Formulierung zur Verwendung nach einem der Ansprüche 1 bis 5, wobei die Verwendung eines oder mehrere der Folgenden bereitstellt:
• eine Verringerung der Häufigkeit, mit der der Hund das Akral-Lick-Granulom leckt, um mindestens 30 %, an dem Tag, der 28 Tage nach Erhalt des Capsaicins liegt, verglichen mit der durchschnittlichen Häufigkeit, mit der der Hund in dem 7-Tage-Zeitraum vor Erhalt des Capsaicins das Akral-Lick-Granulom pro Tag leckt;
• eine Verringerung der Häufigkeit, mit der der Hund am Akral-Lick-Granulom beißt, um mindestens 30 % an dem Tag, der 28 Tage nach dem Erhalt des Capsaicins liegt, verglichen mit der durchschnittlichen Häufigkeit, mit der der Hund in dem 7-Tage-Zeitraum vor Erhalt des Capsaicins pro Tag am Akral-Lick-Granulom beißt;
• eine Verringerung der Häufigkeit, mit der der Hund das Akral-Lick-Granulom kratzt, um mindestens 30 % an dem Tag, der 28 Tage nach dem Erhalt des Capsaicins liegt, verglichen mit der durchschnittlichen Häufigkeit, mit der der Hund in dem 7-Tage-Zeitraum vor Erhalt des Capsaicins pro Tag das Akral-Lick-Granulom kratzt; oder
• erneutes Haarwachstum spätestens am 28. Tag nach Erhalt des Capsaicins über mindestens 30 % der topischen Fläche der ursprünglichen Läsion aufgrund des Akral-Lick-Granuloms.

7. Formulierung zur Verwendung nach einem der Ansprüche 1 bis 6, wobei die Verabreichung eines oder mehrere der Folgenden bereitstellt:
• mindestens eine 40%-ige Verringerung des CSOM-Werts (Client-Specific Outcome Measure) am 14. Tag nach Erhalt von Capsaicin im Vergleich zum CSOM-Wert am Tag, der 7 Tage vor dem Erhalt von Capsaicin liegt; oder
• mindestens eine 20%-ige Verringerung des CSOM-Score am Tag 28 nach Erhalt von Capsaicin im Vergleich zu dem CSOM-Score am Tag, der 7 Tage vor dem Erhalt von Capsaicin liegt.

8. Formulierung zur Verwendung nach einem der Ansprüche 1 bis 7, ferner **gekennzeichnet durch** eine Linderung des Juckreizes aufgrund des Akral-Lick-Granuloms für eine Dauer von mindestens 6 Wochen.

9. Formulierung zur Verwendung nach einem der Ansprüche 1 bis 8, ferner **gekennzeichnet durch** eine Schmerzlinderung aufgrund des Akral-Lick-Granuloms für eine Dauer von mindestens 6 Wochen.

10. Formulierung zur Verwendung nach einem der Ansprüche 1 bis 9, ferner **gekennzeichnet durch** Bereitstellen einer Deaktivierung einer Nervenfaser, ausgewählt aus der Gruppe, bestehend aus einer C-Faser und einer A-Delta-Faser, die sich jeweils in oder proximal eines Akral-Lick-Granuloms im Hund für eine Dauer von mindestens 4 Wochen befinden.

11. Formulierung zur Verwendung nach einem der Ansprüche 1 bis 10, wobei die Verwendung die Verabreichung eines Anästhetikums vor dem Capsaicinoid umfasst.

12. Formulierung zur Verwendung nach Anspruch 11, wobei das Anästhetikum ein Mittel ist, das eine allgemeine Sedierung des Hundes verursacht.

13. Formulierung zur Verwendung nach Anspruch 11, wobei das Anästhetikum ein Hundeanalgetikum ist, das proximal zum Granulom verabreicht wird.

14. Formulierung zur Verwendung nach Anspruch 13, wobei das Hundeanalgetikum Lidocain oder ein pharmazeutisch verträgliches Salz davon ist.

## Revendications

1. Formulation injectable de capsaïcine liquide destinée à être utilisée dans le cadre du traitement d'un granulome de léchage des extrémités chez un chien, laquelle formulation injectable de capsaïcine liquide est destinée à être administrée au chien par de multiples injections sous-cutanées dans le tissu à proximité de la limite inférieure du granulome, dans laquelle, après la première injection sous-cutanée de la formulation dans le tissu à proximité de la limite inférieure du granulome, toute injection sous-cutanée ultérieure de la formulation dans le tissu à proximité de la limite inférieure du granulome est effectuée à un emplacement latéralement éloigné de toute injection antérieure de la formulation dans le tissu à proximité de la limite inférieure du granulome, pour ainsi disperser la capsaïcine dans toute la zone sous le granulome de léchage des extrémités à proximité de la limite entre le tissu sain et le granulome ; dans laquelle
l'administration délivre de la capsaïcine dans une quantité allant d'environ 1,2 µg à environ 1,8 µg de capsaïcine par millimètre carré de surface topique du granulome de léchage des extrémités ;
la formulation injectable de capsaïcine liquide contient de la capsaïcine dans une quantité allant d'environ 0,3 mg/ml à environ 0,5 mg/ml ; et
la capsaïcine comprend plus d'environ 95 % de trans-capsaïcine.

2. Formulation à utiliser selon la revendication 1, dans laquelle l'utilisation délivre de la capsaïcine dans une quantité d'environ 1,5 µg de capsaïcine par millimètre carré de surface topique du granulome de léchage des extrémités.

3. Formulation à utiliser selon la revendication 1 ou 2, dans laquelle les multiples injections sous-cutanées de la formulation injectable de capsaïcine liquide dans un tissu à proximité de la limite inférieure du granulome sont des injections de la formulation injectable de capsaïcine liquide dans un tissu sain à proximité de la limite inférieure du granulome.

4. Formulation à utiliser selon la revendication 3, dans laquelle l'utilisation comprend en outre l'injection d'une formulation injectable de capsaïcine liquide dans un tissu de granulome de léchage des extrémités.

5. Formulation à utiliser selon l'une quelconque des revendications 1 à 3, dans laquelle l'utilisation délivre d'environ 0,3 ml à environ 5 ml de la formulation.

6. Formulation à utiliser selon l'une quelconque des revendications 1 à 5, dans laquelle l'utilisation fournit un ou plusieurs des éléments suivants :
• une réduction d'au moins 30 % du nombre de fois où le chien s'est mis à lécher le granulome de léchage des extrémités 28 jours après avoir reçu la capsaïcine par rapport au nombre moyen de fois où le chien s'est mis à lécher le granulome de léchage des extrémités par jour pendant la période de 7 jours avant de recevoir la capsaïcine ;
• une réduction d'au moins 30 % du nombre de fois où le chien s'est mis à mordre le granulome de léchage des extrémités 28 jours après avoir reçu la capsaïcine par rapport au nombre moyen de fois où le chien s'est mis à mordre le granulome de léchage des extrémités par jour pendant la période de 7 jours avant de recevoir la capsaïcine ;
• une réduction d'au moins 30 % du nombre de fois où le chien s'est mis à gratter le granulome de léchage des extrémités 28 jours après avoir reçu la capsaïcine par rapport au nombre moyen de fois où le chien s'est mis à gratter le granulome de léchage des extrémités par jour pendant la période de 7 jours avant de recevoir la capsaïcine ; ou
• une repousse des poils sur au moins environ 30 % de la surface topique de la lésion d'origine due au granulome de léchage des extrémités vingt-huit jours après avoir reçu la capsaïcine.

7. Formulation à utiliser selon l'une quelconque des revendications 1 à 6, dans laquelle l'administration fournit un ou plusieurs des éléments suivants :
• une réduction d'au moins 40 % du score de mesure avec critère spécifique au client (CSOM) 14 jours après avoir reçu de la capsaïcine par rapport au score CSOM observé 7 jours avant de recevoir la capsaïcine ; ou
• une réduction d'au moins 20 % du score CSOM 28 jours après avoir reçu de la capsaïcine par rapport au score CSOM observé 7 jours avant de recevoir la capsaïcine.

8. Formulation à utiliser selon l'une quelconque des revendications 1 à 7, **caractérisée en outre par** le fait de procurer un soulagement des démangeaisons dues au granulome de léchage des extrémités pendant une durée d'au moins 6 semaines.

9. Formulation à utiliser selon l'une quelconque des revendications 1 à 8, **caractérisée en outre par** le fait de procurer un soulagement de la douleur due au granulome de léchage des extrémités pendant une durée d'au moins 6 semaines.

10. Formulation à utiliser selon l'une quelconque des revendications 1 à 9, **caractérisée en outre par** la désactivation d'une fibre nerveuse choisie dans le groupe constitué par une fibre C et une fibre A-delta, chacune étant située dans ou à proximité d'un granulome de léchage des extrémités chez le chien, pendant une durée d'au moins 4 semaines.

11. Formulation à utiliser selon l'une quelconque des revendications 1 à 10, dans laquelle l'utilisation comprend l'administration, avant le capsaïcinoïde, d'un agent anesthésique.

12. Formulation à utiliser selon la revendication 11, dans laquelle l'agent anesthésique est un agent qui provoque une sédation générale du chien.

13. Formulation à utiliser selon la revendication 11, dans laquelle l'agent anesthésique est un analgésique canin administré à proximité du granulome.

14. Formulation à utiliser selon la revendication 13, dans laquelle l'analgésique canin est la lidocaïne ou un sel pharmaceutiquement acceptable de celle-ci.
